# EUROPEAN PATENT APPLICATION

(11) **EP 4 552 634 A1**
(43) Date of publication of application: **14.05.2025**
(21) Application number: 23835845.1
(22) Date of filing: 05.07.2023
(51) Int. Cl.: A61K 9/16, A61K 38/26, A61P 3/10, A61P 3/04, A61P 1/16, A61P 25/28, A61P 9/12

(54) **SUSTAINED RELEASE-MICROSPHERE FORMULATION COMPRISING SEMAGLUTIDE OR PHARMACEUTICALLY ACCEPTABLE SALT THEREOF AND PREPARATION METHOD THEREFOR**

(30) Priority: 05.07.2022 KR 20220082383; 01.09.2022 KR 20220110883; 30.01.2023 KR 20230012166; 08.05.2023 KR 20230059478
(71) Applicant: G2GBIO, Inc., Cheongju-si, Chungcheongbuk-do 28161 (KR)
(72) Inventor: LEE, Juhwa, Cheongju-si, Chungcheongbuk-do 28222 (KR); KIM, Hyemin, Cheongju-si, Chungcheongbuk-do 28222 (KR); MOON, Sungwoong, Daejeon 34046 (KR); LEE, Jinwoo, Sejong 30144 (KR); NA, Yongha, Cheongju-si, Chungcheongbuk-do 28118 (KR); JEONG, Heon, Cheongju-si, Chungcheongbuk-do 28222 (KR); LEE, Juhan, Daejeon 35218 (KR); SEOL, Eunyoung, Daejeon 34080 (KR); LEE, Heeyong, Daejeon 34032 (KR)
(74) Representative: Ricker, Mathias
(86) International application number: PCT/KR2023/009535
(87) International publication number: WO 2024/010379

(57) **Abstract**

The present disclosure relates to a pharmaceutical composition useful for the prevention or treatment of diabetes, preservation of beta-cell function, hypertension, hyperlipidemia, obesity, non-alcoholic steatohepatitis, or neurodegenerative diseases such as Alzheimer's disease and Parkinson's disease, which includes a sustained-release microsphere containing semaglutide or a pharmaceutically acceptable salt thereof, a bioavailability enhancer and a biodegradable polymer, so that the pharmaceutical composition do not have a high initial burst of drug, contain a high content of drug relative to the particle size and have a high bioavailability, and thus, can minimize pain and inflammatory response of patient that may occur when administered to the human body.

## Description

### [TECHNICAL FIELD]

The present disclosure relates to a sustained-release microsphere containing semaglutide or a pharmaceutically acceptable salt thereof with enhanced bioavailability. a pharmaceutical composition comprising the sustained-release microsphere containing a high content of semaglutide, and a method for preparing the sustained-release microsphere.

### [BACKGROUND ART]

Glucagon-like peptide-1 (GLP-1) is a peptide derived from pre-proglucagon which is a 158 amino acid precursor polypeptide that are differentially processed in the tissues to form a number of different proglucagon-derived peptides, including glucagon, glucagon-like peptide-1 (GLP-1), glucagon-like peptide-2 (GLP-2) and oxyntomodulin (OXM). These molecules are involved in a wide variety of physiological functions, including glucose homeostasis, insulin secretion, gastric emptying and intestinal growth, as well as regulation of food intake. GLP-1 is produced as a 37-amino acid peptide that corresponds to amino acids 72 to 108 of pre-proglucagon (pre-proglucagon 92 to 128). GLP-1(7-36) amide or GLP-1(7-37) acid is a biologically active form of GLP-1 that exhibits essentially equivalent activity at the GLP-1 receptor. GLP-1 and GLP-1 analogues acting as agonists at the GLP-1 receptor have been shown to provide effective glycemic control, for example for treating patients with type 2 diabetes, and additionally provide weight loss effects, preservation of β-cell function, and reduction of hypertension, hypoglycemia and/or hyperlipidemia. Certain GLP-1 analogues, including Byetta^{®} Bydureon^{®} & Bydurepn PEN^{®} & Bydureon BCise^{®} (exenatide), Ozempic^{®} & Wegovy^{®} (semaglutide), Victoza^{®} & Saxenda^{®} (liraglutide), Adlyxin^{®} (lixisenatide); Tanzeum^{®} (albiglutide), and Trulicity^{®} (dulaglutide), are commercially available or under development.

GLP-1 agonists such as semaglutide, are peptides, and administration of such peptides is performed mainly through injection due to several barriers such as enzymatic degradation in the gastrointestinal tract and intestinal mucosa, insufficient absorption from the intestinal mucosa, and first pass metabolism in the liver. Recently, oral administration agent has also been commercialized, but its bioavailability is much lower compared to injection formulations, and requires significantly higer doses. Furthermore, dosage forms containing semaglutide are formulated to be directly administered by patients themselves (self-administered) for sustainable management of obesity or diabetes, so that is very important to regulate pain, inflammatory response, etc. that may occur at the administration site.

On the other hand, a technique is known in which semaglutide is encapsulated in microparticles made of biodegradable polymers for long-term release. In this case, due to the low bioavailability of semaglutide encapsulated in the microspheres or the low content of drug in the microspheres, a large number of microspheres must be administered to exhibit long-term effective pharmacological effects. However, a large number of microspheres in vivo are difficult to administer subcutaneously, , making it difficult for the patient to directly administer (self-administer) the drug, can cause severe pain and inflammatory response at the administration site are extremely high.

### [DETAILED DESCRIPTION OF THE INVENTION]

### [Technical Problem]

The present disclosure has been designed to solve the above problems, and an object of the present disclosure is to provide a pharmaceutical composition comprising a sustained-release microsphere consisting of semaglutide or a pharmaceutically acceptable salt thereof, a bioavailability enhancer, and a biodegradable polymer having a high bioavailability of semiglutide and long-term stable drug release properties, and a pharmaceutical composition comprising a sustained-release microsphere consisting of semaglutide or a pharmaceutically acceptable salt thereof and a biodegradable polymer and exhibiting stable drug release properties with a high drug content and a low initial release, and a method for preparing the sustained-release microsphere.

### [Technical Solution]

The present disclosure is described in detail below.

In the present disclosure, the term "at least one' refers to the number corresponding to one or more. In the present disclosure, when the number of any component is one or more, it may preferably be one, two or more, three or more, one to three, or one to two, but is not limited thereto.

In order to achieve the above object, in one aspect of the present disclosure, there is provided a pharmaceutical composition comprising a semaglutide sustained-release microsphere containing a biodegradable polymer, wherein the content of semaglutide is at least 8 wt% as semaglutide based on the total weight of the microspheres, and wherein a bioavailability enhancer is contained in an amount of 2.5wt% to 250 wt% based on the weight of semaglutide.

In another aspect of the present disclosure, there is provided a pharmaceutical composition for preventing or treating diabetes, type 2 diabetes, preservation of beta-cell function, obesity, non-alcoholic steatohepatitis, or neurodegenerative disease, comprising a sustained-release microsphere consisting of semaglutide or a pharmaceutically acceptable salt thereof, an initial-release inhibitor and a biodegradable polymer, wherein the semaglutide or a pharmaceutically acceptable salt thereof is contained in an amount of 8 wt% or more as semaglutide based on the total weight of the microspheres, and the initial-release inhibitor is contained at 5ppm to 2,000 ppm.

In another aspect, the biodegradable polymer may be at least one selected from the group consisting of: a polymer selected from the group consisting of polylactide (PLA), polyglycolide (PGA), polylactide-co-glycolide (PLGA) which is a copolymer of lactide and glycolide, polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and a block copolymer of poly(butylene succinate lactide) (PBSLA); copolymers or simple mixtures of two or more thereof; a copolymer of the polymer with polyethylene glycol (PEG); and at least one selected from the group consisting of polymer-sugar complexes in which the polymer or the copolymer is conjugated to sugar.

In another aspect, the bioavailability enhancer may be at least one selected from the group consisting of: sodium decanoate, disodium phosphate, choline, meglumine, basic aluminum carbonate, dihydroxyaluminum sodium carbonate, ammonium phosphate, histidine, HEPES, HEPPS, spermine, spermidine, putrescine, methylene blue, proline, sugar, glycerol, surfactant, arginine, glycine, guanidine hydrochloride, urea, sodium chloride, potassium chloride, triethylamine, ethanolamine, triethanolamine, ethylenediamine, poloxamer, benzathine, procaine, lidocaine, bupivacaine, ropivacaine, oxytetracycline, sunitinib, rhizolutin, benzofuran, magnesium carbonate, magnesium hydroxide, magnesium oxide, magnesium trisilicate, zinc carbonate, zinc hydroxide, zinc phosphate, aluminum hydroxide, aluminum phosphate, dihydroxyaluminum aminoacetate, calcium phosphate, calcium hydroxide, magaldrate, and benzofuran derivatives.

In another aspect, the pharmaceutically acceptable salt of semaglutide are sodium salt, acetate, benzoate, hydroxynaphthoate, napadisilate, or pamoate of semaglutide.

In another aspect, poly(lactide-co-glycolide), polyglycolide or polylactide among the biodegradable polymers has an intrinsic viscosity of 0.16 dL/g to 1.7 dL/g.

In another aspect, the microspheres containing the semaglutide or a pharmaceutically acceptable salt thereof and a bioavailability enhancer may have an average particle size of 5 µm to 100 µm.

In another aspect, the microspheres containing the semaglutide or a pharmaceutically acceptable salt thereof and a bioavailability enhancer may have a span value of 1.5 or less.

In another aspect, the weight of the microspheres containing the semaglutide or a pharmaceutically acceptable salt thereof and a bioavailability enhancer may be 20 to 1,000 mg, 20 mg to 800 mg, 20 mg to 600 mg, 20 mg to 400 mg, 20 mg to 200 mg, 20 mg to 100 mg, 30 mg to 1,000 mg, 30 mg to 800 mg, 30 mg to 600 mg, 30 mg to 400 mg, 30 mg to 200 mg, 30 mg to 100 mg, 40 mg to 1,000 mg, 40 mg to 800 mg, 40 mg to 600 mg, 40 mg to 400 mg, 40 mg to 200 mg, 40 mg to 100 mg, 50 mg to 1,000 mg, 50 mg to 800 mg, 50 mg to 600 mg, 50 mg to 400 mg, 50 mg to 200 mg, or 50 mg to 100 mg.

In another aspect, one or more release controlling agents selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, naphadicylic acid, naphthalene sulfonic acid and pamoic acid may be further comprised.

In another aspect, the microspheres may contain 1 to 100 mg/kg of Na.

In another aspect, the microspheres may contain 10 to 500 mg/kg of P.

In another aspect of the present disclosure, there is provided a method for preparing a sustained-release microsphere containing semaglutide or a pharmaceutically acceptable salt thereof and a bioavailability enhancer.

In another aspect, the preparation method may use a continuous phase containing an initial-release inhibitor.

In another aspect, the initial-release inhibitor may be a salt that is dissolved in the continuous phase to maintain the pH of the continuous phase at 7.0 or higher, when the microspheres containing semaglutide is prepared.

In another aspect, the initial-release inhibitor may be at least one substance that forms one or more anions selected from alkali metal, alkaline earth metal or ammonium phosphate salt, phosphide salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt and nitride salt. In another aspect, the initial-release inhibitor may be at least one selected from the group consisting of disodium phosphate, dipotassium phosphate, and diammonium phosphate.

In another aspect, there is provided a pharmaceutical composition comprising a sustained-release microsphere containing a biodegradable polymer, wherein the content of semaglutide is 8 wt% or more as semaglutide based on the total weight of the microspheres, and the initial release of the drug is less than 10%, and a method preparing the same.

### [Advantageous Effects]

A sustained-release pharmaceutical composition comprising semaglutide or a pharmaceutically acceptable salt thereof and a bioavailability enhancer according to Preparation Examples of the present disclosure can contain a high amount of drug relative to the particle size, and have a high bioavailability when administered into the body, thereby reducing the dosage while exhibiting long-lasting effects. Thus, it can minimize pain and inflammatory response of patient that may occur when administered.

### [BRIEF DESCRIPTION OF THE DRAWINGS]

FIG. 1 is a graph showing the changes in blood drug concentration over time after administering semaglutide-containing microspheres prepared in Preparation Example 12 to rats.

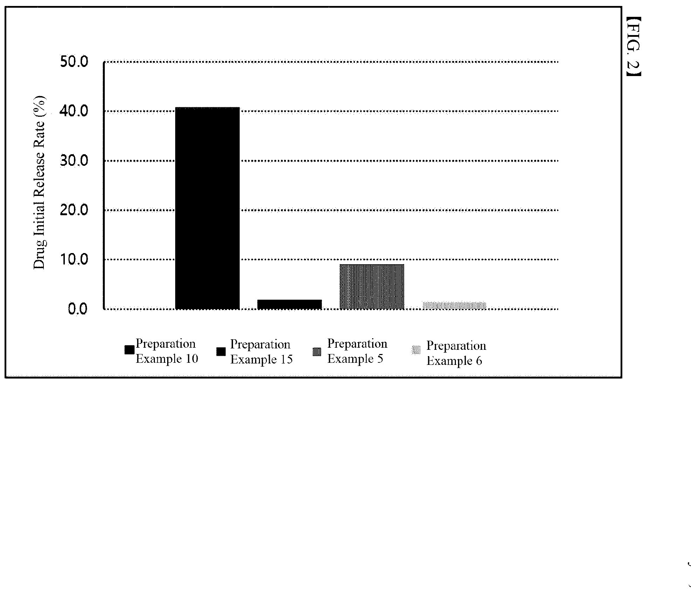

FIG. 2 is a graph showing the initial release of semaglutide-containing microspheres prepared in Preparation Examples 5, 6, 10, and 15.
FIG. 3A is a scanning electron microscope (SEM) photograph showing a cross-sectional surface of microspheres prepared using only 0.1 (w/v)% of PVA in the continuous phase.
FIG. 3B is a scanning electron microscope photograph showing a cross-sectional surface of microspheres prepared using 0.1 (w/v)% of PVA and 2 (w/v)% of Na₂HPO₄ in the continuous phase.

### [DETAILED DESCRIPTION OF THE EMBODIMENTS]

Hereinafter, the present disclosure will be described in detail.

The present disclosure includes semaglutide or a pharmaceutically acceptable salt thereof as an active ingredient.

Semaglutide is GLP-1 receptor agonist N^{6.26}-{18-[N-(17-carboxy-heptadecanoyl)-L-γ-glutamyl]-10-oxo3,6,12,15-tetraoxa-9,18-diazaoctadecanoyl}-[8-(2-amino-2-propanoic acid), 34-L-arginine]human glucagon-like peptide 1(7-37), and may also be called N-epsilon26-[2-(2-{2-[2-(2-{2-[(S)-4-carboxy-4-(17-carboxyheptadecanoylamino)butyrylamino]ethoxy}ethoxy)acetylamino]ethoxy}ethoxy) acetyl][Aib8,Arg34]GLP-1-(7-37). The structure of semaglutide is represented by the following Chemical Formula 1.

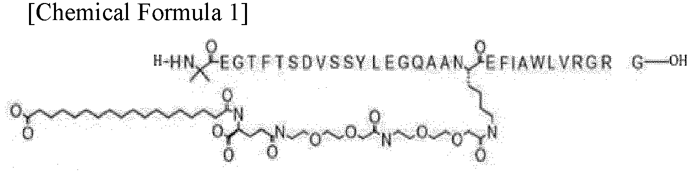

Such semaglutide can be prepared as described in Preparation Example 4 of International Patent Publication WO2006/097537, and commercially available semaglutide can also be used.

Semaglutide may be present in the form of a salt, particularly a pharmaceutically acceptable salt. As the salt, a salt commonly used in the art can be used without limitation. The term "pharmaceutically acceptable salt" as used herein refers to any organic or inorganic addition salt of the compound whose concentration exhibits relatively non-toxic and harmless effective actions to a patient, and whose side effects do not decrease the beneficial efficacy of the above compound. Specific examples include, but are not limited to, the sodium salt, acetate, benzoate, hydroxynaphthoate, naphadisylate, or pamoate of semaglutide.

Semaglutide or a pharmaceutically acceptable salt thereof, which is an active ingredient of the present disclosure, may be in various forms, for example, amorphous or crystalline.

In the present disclosure, "bioavailability enhancer" is a substance that is encapsulated in microparticles together with the active ingredient, semaglutide or a pharmaceutically acceptable salt thereof to enhance the bioavailability. Specifically, when it is contained in the sustained-release microsphere containing semaglutide or a pharmaceutically acceptable salt thereof and the microsphere is administered into the body, it refers to a substance in which the area under the blood concentration-time curve (AUC) of semaglutide increases compared to that of microspheres that do not contain a "bioavailability enhancer." More specifically, when the sustained-release microsphere is administered to rats, it refers to a substance that the area under the blood concentration-time curve of semaglutide (AUCₜₒₜₐₗ/(mg/kg)) when administering 1 mg/kg as semaglutide appears at 600 ng*day/mL or more, preferably 1,000 ng*day/mL or more, more preferably 1,300 ng*day/mL or more.

In a specific aspect, the bioavailability enhancer may be at least one selected from the group consisting of: sodium decanoate, disodium phosphate, choline, meglumine, basic aluminum carbonate, dihydroxyaluminum sodium carbonate, ammonium phosphate, histidine, HEPES(hydroxyethyl piperazine ethane sulfonic acid) HEPPS(4-(2-hydroxyethyl)piperazine-1-propanesulfonic acid), spermine, spermidine, putrescine, methylene blue, proline, sugar, glycerol, surfactant, arginine, glycine, guanidine hydrochloride, urea, sodium chloride, potassium chloride, triethylamine, ethanolamine, triethanolamine, ethylenediamine, poloxamer, benzathine, procaine, lidocaine, bupivacaine, ropivacaine, oxytetracycline, sunitinib, rhizolutin, benzofuran, magnesium carbonate, magnesium hydroxide, magnesium oxide, magnesium trisilicate, zinc carbonate, zinc hydroxide, zinc phosphate, aluminum hydroxide, aluminum phosphate, dihydroxyaluminum aminoacetate, calcium phosphate, calcium hydroxide, magaldrate, and benzofuran derivatives.

More specifically, the bioavailability enhancer may be at least one selected from the group consisting of sodium decanoate, disodium phosphate, choline, histidine, HEPES, glycerol, surfactants such as Tween 80 and Span 80, arginine, poloxamer, benzathine and bupivacaine, but is not limited thereto.

More specifically, the bioavailability enhancer may be at least one selected from the group consisting of sodium decanoate, disodium phosphate, poloxamer F127 and benzathine, but is not limited thereto.

In a specific embodiment, the bioavailability enhancer may be included in an amount of 2.5 wt% to 250 wt%, 2.5 wt% to 200 wt%, 2.7 wt% to 150 wt%, or 2.7 wt% to 100 wt% based on the weight of semaglutide, but is not limited thereto.

The pharmaceutical composition according to the present disclosure has an area under the blood concentration-time curve of semaglutide up to 24 hours after administration (AUC₀₋₂₄ₕᵣ) of 20% or less, 10% or less, 5% or less, 0.1 to 20%, 1 to 10%, or 1 to 5% relative to the area under the whole blood concentration-time curve (AUCₜₒₜₐₗ).

The pharmaceutical composition according to the present disclosure may have an area under the blood concentration-time curve of semaglutide from administration to the administration interval day (AUC_{0-QXM}) of 95% or less, 90% or less, 85% or less, 0.1 to 95%, 1 to 95%, 10 to 95%, 1 to 90%, 10 to 90%, 20 to 95%, 30 to 95%, 40 to 95%, 50 to 95%, 60 to 95%, 70 to 95%, 80 to 95%, or 70 to 85%, relative to the area under the whole blood concentration-time curve (AUCₜₒₜₐₗ).

Moreover, the pharmaceutical composition according to the present disclosure may be a pharmaceutical composition containing sustained-release microspheres in which the semaglutide content is 12 wt% or more as semaglutide based on the total weight of microspheres, and the initial release of the drug is less than 10% within 24 hours.

The biodegradable polymer contained in the semaglutide sustained-release microspheres contained in the pharmaceutical composition according to the present disclosure is at least one selected from the group consisting of: a polymer selected from the group consisting of polylactide (PLA), polyglycolide (PGA), polylactide-co-glycolide (PLGA), which is a copolymer of lactide and glycolide, polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); copolymers or simple mixtures of two or more thereof; a copolymer of the above polymer with polyethylene glycol (PEG); and a polymer-sugar conjugate in which the above polymer or the copolymer is conjugated to sugar.

In one specific aspect, the pharmaceutical composition according to the present disclosure may include microspheres containing two or more types of the above-mentioned biodegradable polymers. In another specific embodiment, the pharmaceutical composition according to the present disclosure may contain two or more types of microspheres each containing one or more types of two or more polymers selected from the above-mentioned biodegradable polymers.

The two or more polymers selected from the group consisting of poly-lactide-co-glycolide and polylactide polymers have an intrinsic viscosity of 0.16 dL/g to 1.7 dL/g, 0.2 dL/g to 1.3 dL/g, or 0.24 dL/g to 1.2 dL/g, but is not limited thereto.

The intrinsic viscosity of poly-lactide-co-glycolide or polylactide used in the present disclosure refers to an intrinsic viscosity measured at a concentration of 0.1% (w/v) in chloroform at 25°C using an Ubbelohde viscometer. If the intrinsic viscosity of poly-lactide-co-glycolide or polylactide is less than 0.16 dL/g, the molecular weight of the polymer is not sufficient and thus, it is difficult to exhibit the sustained-release effect of the semaglutide or its pharmaceutically acceptable salt. If the intrinsic viscosity exceeds 1.7 dL/g, it may exhibit the effect of excessively delaying the release of the semaglutide or its pharmaceutically acceptable salt. Further, when preparing the microsphere using a polymer having a high intrinsic viscosity, there is a problem in that an excessive amount of a production solvent must be used due to the high viscosity of the polymer, and it is difficult to prepare a reproducible microsphere. Examples of commercially available polymers having the above-mentioned properties include RG502H, RG503H, RG504H, RG502, RG503, RG504, RG653H, RG752H, RG752S, RG755S, RG750S, RG757S, RG858S, R202H, R203H, R205H, R202S, R203S, R205S, R206S, and R207S (Resomer^{®} series available from Evonik), and PDL 02A, PDL 02, PDL 04, PDL 05, PDLG 7502A, PDLG 7502, PDLG 7507, PDLG 5002A, PDLG 5002, PDLG 5004A and PDLG 5004 (available from Corbion Purac), and the like.

The content of the biodegradable polymer in the sustained-release microsphere containing semaglutide or a pharmaceutically acceptable salt thereof and a bioavailability enhancer according to the present disclosure may be 60 to 95 wt%, 65 to 93 wt%, or 70 to 90 wt% based on the total weight of the microsphere, but is not limited thereto.

The content of semaglutide or a pharmaceutically acceptable salt thereof in the sustained-release microsphere containing semaglutide or a pharmaceutically acceptable salt thereof and a bioavailability enhancer of the present disclosure is preferably 8 wt% or more, 12 wt% or more, 14 wt% or more, 37 wt% or less, 35 wt% or less, or 33 wt% or less as semaglutide based on the total weight of the microsphere. If the content of semaglutide or its pharmaceutically acceptable salt in the microsphere is less than 8 wt% as semaglutide, the amount of the polymer used is too high relative to the drug, which may reduce the bioavailability of of semaglutide or its pharmaceutically acceptable salt. If the content is excessively high, there is a problem that the initial release of semaglutide or a pharmaceutically acceptable salt thereof may increase, which is not preferable.

The microspheres containing semaglutide or a pharmaceutically acceptable salt thereof and a bioavailability enhancer according to the present disclosure preferably have a uniform particle distribution profile with an average particle size of 5 µm to 100 µm, 5 to 90 µm, 5 µm to 80 µm, 10µm to 90 µm, 10 µm to 80 µm, 15 µm to 100 µm, 15 µm to 90 µm, 15 µm to 80µm, 70 µm to 100 µm, 70 µm to 90 µm, 70 µm to 80 µm, 60 µm to 100 µm, 60 µm to 80 µm, 60 µm to 70 µm, 20 µm to 90 µm, 20 µm to 70 µm, 20 µm to 60 µm, 30 µm to 80 µm, 30 µm to 60 µm, 40 µm to 70 µm, 40 µm to 50 µm, 30 µm to 40 µm, 20 µm to 30 µm, 5 µm to 30 µm, 5 µm to 20 µm, 10 µm to 20 µm, or 5 µm to 10 µm. The term "average particle size" as used herein refers to a median diameter as the particle size corresponding to 50% of the volume % in the particle size distribution curve, and is represented by D50 or D(v, 0.5).

If the average particle size of the microspheres containing semaglutide or its pharmaceutically acceptable salt and a bioavailability enhancer is less than 5 µm, the semaglutide or its pharmaceutically acceptable salt drug from the microsphere is released too rapidly, which is not preferable. If the average particle size is greater than 100 µm, an injection needle becomes too thicker when administered in a human body, which causes pain during injection, or leak of drug at the injection site after injection, which is not preferable.

Preferably, the microparticles containing semaglutide or its pharmaceutically acceptable salt and a bioavailability enhancer of the present disclosure has a uniform particle size distribution. The microparticles having a uniform particle size distribution which contain semaglutide or its pharmaceutically acceptable salt and a bioavailability enhancer can be administered in a more accurate amount with less deviation during injection than the microspheres having non-uniform particle size distribution. Preferably, the span value of the microsphere containing semaglutide or its pharmaceutically acceptable salt and a bioavailability enhancer of the present disclosure is 1.5 or less. More preferably, the span value is 1.2 or less. More specifically, the span value may be 1.5 or less, 1.2 or less, 0.1 to 1.5, 0.3 to 1.5, 0.5 to 1.5, 0.1 to 1.0, 0.4 to 1.0, 0.6 to 1.0, 0.2 to 0.8, or 0.4 to 0.8. The span value as used herein is an index indicating the uniformity of the particle size of the microsphere, and means a value determined by the formula of span value=(Dv0.9-Dv0.1)/Dv0.5. Here, Dv0.1 means a particle size corresponding to 10% of the volume% in the particle size distribution curve of the microsphere, Dv0.5 refers to a particle size corresponding to 50% of the volume % in the particle size distribution curve of the microsphere, and Dv0.9 means a particle size corresponding to 90% of the volume % in the particle size distribution curve of the microsphere.

Since the sustained-release microsphere containing semaglutide or its pharmaceutically acceptable salt and a bioavailability enhancer of the present disclosure is administered by an injection route, for example, subcutaneous injection, and can be particularly self-administered, it is preferable that semaglutide is released over a relatively long period of time. Preferably, the sustained-release microsphere in the pharmaceutical composition according to the present disclosure can release semaglutide or a pharmaceutically acceptable salt thereof for 1 month or more, 2 months or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, but are not limited thereto. Further, the sustained-release microsphere containing semaglutide or a pharmaceutically acceptable salt thereof and a bioavailability enhancer of the present disclosure are not particularly limited in this release mode, but when administered in vivo, it is preferable that less than 10%, less than 15%, or less than 20% of semaglutide or a pharmaceutically acceptable salt thereof is released within 24 hours.

Further, in the pharmaceutical composition of the present disclosure, the total amount of the sustained-release microsphere formulation containing semaglutide or a pharmaceutically acceptable salt thereof and a bioavailability enhancer may be 20 to 1,000 mg, 20 mg to 800 mg, 20 mg to 600 mg, 20 mg to 400 mg, 20 mg to 200 mg, 20 mg to 100 mg, 30 mg to 1,000 mg, 30 mg to 800 mg, 30 mg to 600 mg, 30 mg to 400 mg, 30 mg to 200 mg, 30 mg to 100 mg, 40 mg to 1,000 mg, 40 mg to 800 mg, 40 mg to 600 mg, 40 mg to 400 mg, 40 mg to 200 mg, 40 mg to 100 mg, 50 mg to 1,000 mg, 50 mg to 800 mg, 50 mg to 600 mg, 50 mg to 400 mg, 50 mg to 200 mg, or 50 mg to 100 mg. As the sustained-release microspheres containing semaglutide or a pharmaceutically acceptable salt thereof and a bioavailability enhancer in the composition is included within the above range, it has the advantage that the composition according to the present disclosure not only minimizes inflammatory response at the administration site, but also can be self-administered by patients.

The microsphere containing semaglutide or a pharmaceutically acceptable salt thereof and a bioavailability enhancer contained in the composition of the present disclosure may further include a release controlling agent. The material used as the release controlling agent may include, for example, at least one selected from the group consisting of butyric acid, valeric acid, caproic acid, enanthic acid, caprylic acid, pelargonic acid, capric acid, undecylenic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, naphadicylic acid, naphthalene sulfonic acid and pamoic acid, but is not limited thereto. Preferably, the release controlling agent may be hydroxynaphthoic acid, naphadicylic acid, or pamoic acid, but is not limited thereto.

A pharmaceutical composition comprising microspheres containing semaglutide or a pharmaceutically acceptable salt thereof and a bioavailability enhancer according to the present disclosure may be formulated in various forms of preparations, for example, it may be in the form of a known parenteral formulation. Therefore, the pharmaceutical composition according to the present disclosure may further include a thickener, a stabilizer, an isotonic agent, an initial-release inhibitor, a surfactant, an excipient and/or a carrier in addition to the semaglutide or a pharmaceutically acceptable salt thereof and a bioavailability enhancer. The isotonic agent that can be used may be water-soluble excipients or sugars such as mannitol, sucrose, sorbitol, trehalose, lactose, sodium chloride, and the like, and examples of the thickener include carmellose sodium, carboxymethyl cellulose sodium, and povidone. Additionally, sodium monohydrogen phosphate, anhydrous citric acid, sodium hydroxide, sodium chloride, and the like can be used as a buffering agent.

The pharmaceutical composition containing a sustained-release microsphere according to the present disclosure may contain less than a certain amount of an initial-release inhibitor among the microsphere.

In the present disclosure, the initial-release inhibitor is a substance that is included in the continuous phase in order to suppress the rapid release of the active ingredient in the microsphere preparation process, and is characterized by being present in less than a certain amount in the microsphere according to the present disclosure.

In a specific embodiment, the initial-release inhibitor may be included in an amount of 5 to 2000 ppm, preferably 10 to 1500 ppm, more preferably 20 to 1,000 ppm, and most preferably 20 to 500 ppm based on the total weight of the microsphere.

In a specific embodiment, the initial-release inhibitor is a material that can maintain the pH of the continuous phase at 7 or more, more specifically, 7 or more, 7.2 or more, 7.4 or more, or 8.0 or more, 8.5 or more, 9.0 or more, 7.0 to 9.0, or 7.0 to 8.5 when dissolved in the continuous phase, and any material can be used as long as it can form divalent or higher anions in the continuous phase. The initial-release inhibitor may be at least one substance selected from alkali metal, alkaline earth metal or ammonium phosphate salt, phosphide salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt and nitride salt, but is not limited thereto. In a specific embodiment, the initial-release inhibitor may be at least one substance selected from disodium phosphate, dipotassium phosphate, and diammonium phosphate, but is not limited thereto.

The pharmaceutical composition according to the present disclosure can be administered with a therapeutically effective amount of semaglutide, for example, in an amount effective to treat diabetes, specifically type 2 diabetes, preservation of beta cell function, hypertension, hyperlipidemia, obesity, non-alcoholic steatohepatitis, or neurodegenerative diseases such as Alzheimer's disease and Parkinson's disease. The therapeutically effective amount of semaglutide can be assessed by a physician. The pharmaceutical composition of the present disclosure containing semaglutide may be administered once a month to once a quarter of year. In some embodiments, the monthly dosage of the composition according to the present disclosure may be 1 mg to 100 mg, 1 mg to 80 mg, 1 mg to 60 mg, 1 mg to 30 mg, 1 mg to 20 mg, 1 mg to 10 mg, 1 mg to 8 mg, 1 mg to 6 mg, 2 mg to 100 mg, 2 mg to 80 mg, 2 mg to 60 mg, 2 mg to 30 mg, 2 mg to 20 mg, 2 mg to 10 mg, 2 mg to 8 mg, 2 mg to 6 mg, 4 mg to 100 mg, 4 mg to 80 mg, 4 mg to 60 mg, 4 mg to 30 mg, 4 mg to 20 mg, 8 mg to 100 mg, 8 mg to 80 mg, 8 mg to 60 mg, 8 mg to 30 mg, 10 mg to 100 mg, 10 mg to 80 mg, 10 mg to 60 mg, 10 mg to 30 mg, 20 mg to 100 mg, 20 mg to 80 mg, 20 mg to 60 mg, or 20 mg to 30 mg based on semaglutide. The pharmaceutical composition according to the present disclosure comprising microspheres containing semaglutide or a pharmaceutically acceptable salt thereof according to the present disclosure and a bioavailability enhancer may be administered parenterally, for example, by subcutaneous injection. The pharmaceutical composition according to the present disclosure may consist of a drug part containing microspheres containing semaglutide or a pharmaceutically acceptable salt thereof and a bioavailability enhancer and a solvent part used to suspend the microspheres. Further, the pharmaceutical composition may be in the form of a dual chamber syringe containing a drug part in one chamber and a solvent part in another chamber, or a prefilled syringe in which the drug part is suspended in the solvent part. When the drug part is configured in the form of a prefilled syringe in which the drug part is suspended in the solvent part, the solvent part used may be an injectable oil containing medium chain oil, mineral oil, etc.

In one specific embodiment, the sustained-release microspheres containing semaglutide or a pharmaceutically acceptable salt thereof and a bioavailability enhancer contained in the pharmaceutical composition according to the present disclosure have a high drug content compared to the content of the microspheres, suppress the initial excessive release of the drug that can lead to fatal side effects, and have high bioavailability, showing sufficient efficacy as a GLP-1 agonist for the desired period of time, and thus, is useful for the prevention or treatment of diabetes, specifically type 2 diabetes, preservation of beta-cell function, hypertension, hyperlipidemia, obesity, non-alcoholic steatohepatitis, or neurodegenerative diseases such as Alzheimer's disease and Parkinson's disease.

In another aspect of the present invention, there is provided a method for preparing a sustained-release microsphere containing semaglutide or a pharmaceutically acceptable salt thereof and a bioavailability enhancer.

In another aspect of the present invention, there is provided a method of producing sustained-release microspheres having significantly suppressed initial release and containing a high content of semaglutide or a pharmaceutically acceptable salt thereof.

Hereinafter, a method for preparing a sustained-release microsphere injection containing semaglutide or a pharmaceutically acceptable salt thereof and a bioavailability enhancer of the present disclosure will be described in detail.

The sustained-release microsphere injection formulation containing semaglutide or a pharmaceutically acceptable salt thereof and a bioavailability enhancer according to the present disclosure may be prepared using, for example, a "solvent extraction and evaporation method" but not limited thereto.

In one aspect of a specific preparation method of the present disclosure, there is provided a method for preparing a sustained-release microsphere containing semaglutide or a pharmaceutically acceptable salt thereof and a bioavailability enhancer, comprising the steps of:
(a) a step of dissolving semaglutide or a pharmaceutically acceptable salt thereof, a bioavailability enhancer, and one or more biodegradable polymers in one or more organic solvent to prepare a solution (dispersed phase) containing semaglutide or a pharmaceutically acceptable salt thereof, a bioavailability enhancer, and a polymer;
(b) a step of adding the solution containing semaglutide or a pharmaceutically acceptable salt thereof, a bioavailability enhancer, and a polymer prepared in step (a) to an aqueous solution phase (continuous phase) containing a surfactant to prepare an emulsion;
(c) extracting the organic solvent from the dispersed phase in the emulsion state prepared in step (b) into a continuous phase and evaporating the organic solvent to form a microsphere; and
(d) recovering the microsphere from the continuous phase of step (c) to prepare a microsphere containing semaglutide or a pharmaceutically acceptable salt thereof with enhanced bioavailability and a bioavailability enhancer.

In another aspect, the bioavailability enhancer in step (a) is at least one selected from the group consisting of: poloxamer, benzathine, procaine, lidocaine, bupivacaine, ropivacaine, oxytetracycline, sunitinib, rhizolutin and benzofuran.

In one aspect of the present disclosure, there is provided a method for preparing a sustained-release microsphere containing semaglutide or a pharmaceutically acceptable salt thereof and a bioavailability enhancer, comprising the steps of:
(a') dissolving semaglutide or a pharmaceutically acceptable salt thereof and a bioavailability enhancer in an aqueous solution to prepare a primary aqueous solution phase, dissolving one or more biodegradable polymers in an organic solvent to prepare an oil phase, and then mixing the primary aqueous solution and the oil phase to prepare a W/O emulsion (primary emulsion) containing semaglutide or a pharmaceutically acceptable salt thereof, a bioavailability enhancer, and a polymer;
(b') adding the primary emulsion containing semaglutide or a pharmaceutically acceptable salt thereof, a bioavailability enhancer, and a polymer prepared in step (a') to an aqueous solution phase (continuous phase) containing a surfactant to prepare a W/O/W emulsion (secondary emulsion);
(c') extracting the organic solvent from the oil phase in the secondary emulsion state prepared in step (b') into a continuous phase and evaporating the organic solvent to form a microsphere; and
(d') recovering the microsphere from the continuous phase of step (c') to prepare a microsphere containing semaglutide or a pharmaceutically acceptable salt thereof with enhanced bioavailability and a bioavailability enhancer.

In another aspect, the bioavailability enhancer in step (a') is at least one selected from the group consisting of: sodium decanoate, disodium phosphate, choline, meglumine, basic aluminum carbonate, dihydroxyaluminum sodium carbonate, ammonium phosphate, histidine, HEPES, HEPPS, spermine, spermidine, putrescine, methylene blue, proline, sugar, glycerol, surfactant, arginine, glycine, guanidine hydrochloride, urea, sodium chloride, potassium chloride, triethyleneamine, ethanolamine, triethanolamine, and ethylenediamine.

In another aspect of the present disclosure, there is provided a method for preparing a sustained-release microsphere containing semaglutide or a pharmaceutically acceptable salt thereof and a bioavailability enhancer, comprising the steps of:
(a") dissolving semaglutide or a pharmaceutically acceptable salt thereof and one or more biodegradable polymers in an organic solvent, and suspending a bioavailability enhancer in this solution to prepare a suspension (dispersed phase) containing semaglutide or a pharmaceutically acceptable salt thereof, a bioavailability enhancer, and a polymer;
(b") adding the suspension containing semaglutide or a pharmaceutically acceptable salt thereof, a bioavailability enhancer, and a polymer prepared in step (a") to an aqueous solution phase (continuous phase) containing a surfactant to prepare an emulsion;
(c") extracting the organic solvent from the dispersed phase in the emulsion state prepared in step (b") into a continuous phase and evaporating the organic solvent to form a microsphere; and
(d") recovering microspheres from the continuous phase of step (c") to prepare a microsphere containing semaglutide or a pharmaceutically acceptable salt thereof with enhanced bioavailability and a bioavailability enhancer.

In another aspect, the bioavailability enhancer in step (a") is at least one selected from the group consisting of: magnesium carbonate, magnesium hydroxide, magnesium oxide, magnesium trisilicate, zinc carbonate, zinc hydroxide, zinc phosphate, aluminum hydroxide, aluminum phosphate, dihydrxoyaluminum aminoacetate, calcium phosphate, calcium hydroxide, magaldrate, and benzofuran derivatives.

In another aspect, the pH of the continuous phase used in the above preparation methods may be 7 or more.

In the preparation of the sustained-release microsphere containing semaglutide or a pharmaceutically acceptable salt thereof and a bioavailability enhancer according to the present disclosure, in order to suppresses excessive release of the initial semaglutide or a pharmaceutically acceptable salt thereof while containing a high amount of semaglutide or a pharmaceutically acceptable salt thereof relative to the weight of the microparticle, have high bioavailability, and be released at a constant concentration for a desired long period of time, e.g. for one month or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, the following biodegradable polymers can be used, but are not limited thereto. The biodegradable polymer that can be used may be at least one polymer, preferably two or more polymers, selected from the group consisting of: a polymer selected from the group consisting of polylactide (PLA), polyglycolide (PGA), polylactide-co-glycolide (PLGA), which is a copolymer of lactide and glycolide, polydioxanone, polycaprolactone(PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); simple mixtures of two or more thereof; a copolymer of the above polymer with polyethylene glycol (PEG); and a polymer-sugar conjugate in which the polymer or the copolymer is conjugated to sugar. **In** one specific embodiment, in the preparation method according to the present disclosure, poly(lactide-co-glycolide) and/or polylactide polymer can be used as the biodegradable polymer.

In the present disclosure, two or more different types of biodegradable polymers may include two or more polymers having different repeating units constituting the polymer and two or more polymers having different molar ratios of the repeating units when containing two or more repeating units.

As an example, the microsphere may be a mixture of a microsphere containing poly-lactide-co-glycolide and a microsphere containing a polylactide polymer, or may be a microsphere containing both poly-lactide-co-glycolide and polylactide polymer.

Further, as a specific example, when two types of biodegradable polymers different from each other are used, the content ratio of these biodegradable polymers may be 0.5:10 to 10:0.5, 0.5:8 to 8:0.5, 1:10 to 10:1, 1:4 to 4:1, 1:3 to 3:1, or 1:2 to 2:1, but is not limited thereto.

In a more specific embodiment, in order to prepare a sustained-release microsphere containing semaglutide or a pharmaceutically acceptable salt thereof and a bioavailability enhancer according to the present disclosure, when polylactide-co-glycolide is used as two or more types of biodegradable polymers, at least one biodegradable polymer having a viscosity of 0.16 dL/g to 0.45 dL/g may be included.

In step (a), (a') or (a") of the above specific preparation method, the organic solvent used to dissolve one or more biodegradable polymers is one or more organic solvents. Further, as the organic solvent, a mixed organic solvent in which two or more types of organic solvents are mixed can also be used. In a specific embodiment, the mixed solvent may be a mixed solvent of an organic solvent that is miscible with water and an organic solvent that is immiscible with water. In this case, the organic solvent that is immiscible with water is preferably used in an amount of at least 50% (v/v) or more, 60 (v/v)% or more, 50 to 99.9 (v/v)%, 50 to 90 (v/v), 50 to 80 (v/v)%, 50 to 70 (v/v)%, 60 to 90 (v/v)%, or 60 to 80 (v/v)%. By using the water-immiscible property of the organic solvent, the continuous phase containing a surfactant in step (b), (b'), or (b") described below can be homogeneously mixed with the dispersed phase to form an emulsion. The type of organic solvent that dissolves one or more of these biodegradable polymers is not particularly limited, but preferably, a mixed solvent of one or more solvents selected from the group consisting of dichloromethane, chloroform, ethyl acetate, methyl ethyl ketone, acetone, acetonitrile, dimethyl sulfoxide, dimethylformamide, N-methylpyrrolidone, acetic acid, methyl alcohol, ethyl alcohol, propyl alcohol and benzyl alcohol, more preferably, a solvent selected from dichloromethane and ethyl acetate, and one or more organic solvents selected from dimethyl sulfoxide, N-methylpyrrolidone, methyl alcohol, and acetic acid can be used.

The method of homogeneously mixing the dispersed phase and the continuous phase containing the surfactant in step (b), (b') or (b") is not particularly limited, but can be performed using a high-speed stirrer, an in-line mixer, a membrane emulsion method, a microfluidics emulsion method, an ultrasonic mixer, a static mixer, or the like. When forming an emulsion using a high-speed stirrer, an in-line mixer, an ultrasonic mixer, or a static mixer, it is difficult to obtain a uniform emulsion and thus, it is preferable to perform an additional sieving step between steps (c) and (d), between steps (c') and (d'), or between steps (c") and (d"), which will be described later.

The type of surfactant used in step (b), (b') or (b") is not particularly limited, and any surfactant can be used as long as it can assist the dispersed phase to form a stable liquid droplet dispersed phase within the continuous phase. Polyvinyl alcohol can be used as the surfactant.

In step (b), (b') or (b"), the content of the surfactant in the continuous phase containing the surfactant may be 0.01 w/v% to 20 w/v%, preferably 0.03 w/v% to 18 w/v, 0.05 w/v% to 15 w/v%, 0.07 w/v% to 10 w/v%, or 0.1 w/v% to 5 w/v% based on the total volume of the continuous phase containing the surfactant.

If the surfactant content is less than 0.01 w/v%, a dispersed phase or emulsion in the form of droplets may not be formed in the continuous phase, and if the surfactant content exceeds 20 w/v%, fine particles are formed in the continuous phase due to an excess of surfactant, and then it may be difficult to remove the surfactant.

The continuous phase used in step (b), (b') or (b") may be water, and in order to control the initial release of microspheres in which a high content of drug is encapsulated, a substance that forms multivalent anions when dissolved in water may be further included as an initial-release inhibitor.

In step (b), (b') or (b"), the type of initial-release inhibitor is not particularly limited, and can be used without limitation as long as it is any basic salt that can be dissolved in the continuous phase and maintain the pH at 7.0 or higher. As the initial-release inhibitor, at least one substance selected from alkali metal, alkaline earth metal or ammonium phosphate salt, phosphide salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt and nitride salt can be used.

In a specific embodiment, the initial-release inhibitor may be at least one substance selected from the group consisting of disodium phosphate, dipotassium phosphate, and diammonium phosphate, but is not limited thereto.

In step (b), (b') or (b"), the content of the initial-release inhibitor contained in the continuous phase used may be 0.05 w/v% to 20 w/v%, preferably 0.1 w/v% to 15 w/v%, more preferably 0.2 w/v% to 12.5 w/v%, most preferably 0.3 w/v% to 10 w/v% based on the total volume of the continuous phase including the initial-release inhibitor.

If the content of the initial-release inhibitor exceeds 20 w/v%, a phenomenon in which emulsion droplets break up may occur during the preparation of microsphere. Conversely, if the content is lower than 0.05 w/v%, the initial release of a microsphere containing a high content of semaglutide may not be sufficiently suppressed.

When the initial-release inhibitor is included in the continuous phase during the preparation of microspheres, the initial release from microspheres containing 12 (w/w)% or more of semaglutide can be reduced to preferably 10% or less, more preferably 8% or less, most preferably, 5% or less.

The continuous phase used in step (b), (b') or (b") may be water, and in order to regulate the extraction rate of the organic solvent from the dispersed phase in an emulsion state, water containing a part of one or more selected from the group consisting of methyl alcohol, ethyl alcohol, propyl alcohol, and ethyl acetate can be used.

Further, the pH of the continuous phase may be 7.0 or more, 7.2 or more, 7.4 or more, 8.0 or more, 8.5 or more, 9.0 or more, 7.0 to 9.0, or 7.0 to 8.5, but is not limited thereto. When the pH of the continuous phase is adjusted within the above range, the bioavailability of the microsphere containing semaglutide or a pharmaceutically acceptable salt thereof may be further increased.

In step (c), (c') or (c"), when an emulsion containing a dispersed phase in the form of droplets and a continuous phase containing a surfactant is maintained or stirred at a temperature below the boiling point of the organic solvent for a certain period of time, for example 2 to 48 hours, the organic solvent may be extracted into a continuous phase from a semaglutide or its pharmaceutically acceptable salt-bioavailability enhancer-polymer solution in the form of a droplet which is a dispersed phase. A part of the organic solvent extracted into the continuous phase can be evaporated from the surface of the continuous phase. As the organic solvent is extracted and evaporated from the a semaglutide or its pharmaceutically acceptable salt-bioavailability enhancer-polymer solution in the form of droplets, the dispersed phase in the form of droplets can be solidified to form microspheres.

In step (c), (c'), or (c"), in order to further efficiently remove the organic solvent, the temperature of the continuous phase may be heated for a certain period of time. The heating temperature is not limited and can be appropriately adjusted by a person skilled in the art depending on the organic solvent used. For example, when dichloromethane is used as the organic solvent, heat may be applied to maintain the temperature at 30°C or higher, 40°C or higher, 45°C or higher, 30 to 50°C, 40 to 50°C, or 45°C.

In step (d), (d') or (d"), the method of recovering the microspheres containing semaglutide or its pharmaceutically acceptable salt and a bioavailability enhancer may be performed using several known techniques, and for example, a method such as filtration or centrifugation can be used.

Between steps (c) and (d), between steps (c') and (d'), or between steps (c") and (d"), the residual surfactant may be removed through filtration and washing, and then the filtration may be performed again to recover the microspheres.

The washing step for removing the residual surfactant can be generally performed using water, and the washing step may be repeated several times.

Further, as described above, a uniform microsphere can be obtained by additionally using a seiving step between steps (c) and (d) and between steps (c') and (d') or between steps (c") and (d"). A sieving step can be performed using a known technique, and microspheres of small particles and large particles can be filtered using a sieve membrane having different sizes to obtain microspheres of uniform size.

In the preparation method of the present disclosure, after step (d), step (d') or step (d") or after the filtration and washing steps, the obtained microspheres are dried using a conventional drying method to finally obtain the dried microspheres.

For all matters including semaglutide, bioavailability enhancer, biodegradable polymer, and their contents that are not separately defined other than the above, the matters defined for the pharmaceutical composition can be applied as they are.

According to the preparation method of the present disclosure, it is possible to prepare a sustained-release microsphere injection formulation containing semaglutide or a pharmaceutically acceptable salt thereof with high bioavailability in which the drug is maintained at an effective concentration for a desired period of time without rapid temporary release. Further, it is possible to prepare a sustained-release microsphere injection formulation containing semaglutide or a pharmaceutically acceptable salt thereof and a bioavailability enhancer in uniform particles which has good administrability.

### [EXAMPLE]

Hereinafter, the present disclosure will be described in more detail by way of Preparation Examples. However, these Preparation Examples are for illustrative purposes only, and the scope of the present disclosure is not limited thereto.

### Preparation Example

Preparation of biodegradable polymer microspheres containing semaglutide or a pharmaceutically acceptable salt thereof and a bioavailability enhancer

### Preparation Example 1 (w/o/w method): Preparation of biodegradable polymer microsphere containing semaglutide and bioavailability enhancer dissolved in the internal aqueous phase

0.15 g of semaglutide sodium salt as a drug (manufacturer: Chengdu Kaijie Biopharm Co., Ltd, China) and 0.33 g of Poloxamer F127 as a bioavailability enhancer were dissolved in 1.66 g of tertiary distilled water to prepare a primary aqueous phase. The organic phase was prepared by mixing RG653H and RG753H (manufacturer: Evonik, Germany) as biodegradable polymers at a 1:1 weight ratio to make 1.52 g and dissolving them in 20.8 g of dichloromethane (manufacturer: J.T. Baker, USA). The primary emulsion (dispersed phase) was prepared by dispersing the aqueous phase in the organic phase using a homogenizer. As the continuous phase, 2,000 mL of a 0.1% polyvinyl alcohol aqueous solution (viscosity: 4.8~5.8 mPa·s) was used. The continuous phase was connected to an emulsification device equipped with a porous membrane with a diameter of 40 µm, and the prepared dispersed phase was injected into the porous membrane together with the continuous phase. An emulsion having dispersed biodegradable polymer microdroplets containing semaglutide was prepared, and the suspension was placed in a preparation container and stirred at a speed of 200 rpm.

The temperature of the preparation container was maintained at 25°C, and when the injection of the dispersed phase was completed, the organic solvent was removed while maintaining the suspension temperature at 40°C for 3 hours. Subsequently, after the temperature was cooled to 25°C, the microspheres were filtered, treated for removing residual polyvinyl alcohol by using tertiary distilled water, and lyophilized.

### Preparation Example 2 (w/o/w method): Preparation of biodegradable polymer microsphere containing semaglutide and bioavailability enhancer dissolved in the internal aqueous phase and an initial-release inhibitor contained in the continuous phase

0.2 g of semaglutide sodium salt as a drug and 0.02 g of disodium phosphate as a bioavailability enhancer were dissolved in 1.0 g of tertiary distilled water to prepare a primary aqueous phase. The organic phase was prepared by dissolving 0.8 g of PDL 04A (manufacturer: Corbion, Netherlands) as a biodegradable polymer in 6.65 g of dichloromethane. The primary emulsion (dispersed phase) was prepared by dispersing the aqueous phase in the organic phase using a homogenizer. As the continuous phase, 2,000 mL of a 0.1% polyvinyl alcohol aqueous solution containing 2% (w/w) disodium phosphate as an initial-release inhibitor was used. The continuous phase was connected to an emulsification device equipped with a porous membrane with a diameter of 40 µm, and the prepared dispersed phase was injected into the porous membrane together with the continuous phase. An emulsion having dispersed biodegradable polymer microdroplets containing semaglutide was prepared, and the suspension was placed in a preparation container and stirred at a speed of 200 rpm.

The temperature of the preparation container was maintained at 25°C, and when the injection of the dispersed phase was completed, the organic solvent was removed while maintaining the suspension temperature at 40°C for 3 hours. Subsequently, the temperature was cooled to 25°C, the microspheres were filtered, treated for removing residual polyvinyl alcohol by using tertiary distilled water, and lyophilized.

### Preparation Example 3 (w/o/w method): Preparation of biodegradable polymer microsphere containing semaglutide, release controlling agent and bioavailability enhancer dissolved in the internal aqueous phase and initial-release inhibitor contained in the continuous phase

0.2 g of semaglutide free base as a drug (manufacturer: Chengdu Kaijie Biopharm Co., Ltd, China) and 0.02 g each of sodium decanoate and disodium phosphate as a release controlling agent and a bioavailability enhancer were dissolved in 1.8 g of tertiary distilled water to prepare a primary aqueous phase. The organic phase was prepared by dissolving 0.76 g of PDLG 7504A (manufacturer: Corbion, Netherlands) as a biodegradable polymer in 14.4 g of dichloromethane. The primary emulsion (dispersed phase) was prepared by dispersing the aqueous phase in the organic phase using a homogenizer. As the continuous phase, 2,000 mL of a 0.1% polyvinyl alcohol aqueous solution containing 2% (w/w) disodium phosphate as an initial-release inhibitor was used. The continuous phase was connected to an emulsification device equipped with a porous membrane with a diameter of 40 µm, and the prepared dispersed phase was injected into the porous membrane together with the continuous phase. An emulsion having dispersed biodegradable polymer microdroplets containing semaglutide was prepared, and the suspension was placed in a preparation container and stirred at a speed of 200 rpm.

The temperature of the preparation container was maintained at 25°C, and when the injection of the dispersed phase was completed, the organic solvent was removed while maintaining the suspension temperature at 40°C for 3 hours. Subsequently, the temperature was cooled to 25°C, the microspheres were filtered, treated for removing residual polyvinyl alcohol by using tertiary distilled water, and lyophilized.

### Preparation Example 4 (o/w method): Preparation of biodegradable polymer microsphere containing semaglutide and bioavailability enhancer dissolved in the organic phase and an initial-release inhibitor contained in the continuous phase

The dispersed phase was prepared by mixing 0.79 g of PDLG 7504A as a biodegradable polymer, 0.2 g of semaglutide free base as a drug and 0.006 g of benzathine as a bioavailability enhancer and thoroughly stirring the mixture using 19.9 g of a mixed solvent of dichloromethane, glacial acetic acid (manufacturer: Daejeong, Korea) and methanol (manufacturer: Daejeong, Korea) (weight ratio of about 16:4:1) for 30 minutes or more. As the continuous phase, 2,000 mL of a 0.1% polyvinyl alcohol aqueous solution containing 2% (w/w) disodium phosphate as an initial-release inhibitor was used. The continuous phase was connected to an emulsification device equipped with a porous membrane with a diameter of 40 µm, and the prepared dispersed phase was injected into the porous membrane together with the continuous phase. An emulsion having dispersed biodegradable polymer microdroplets containing semaglutide was prepared, and the suspension was placed in a preparation container and stirred at a speed of 200 rpm.

The temperature of the preparation container was maintained at 25°C, and when the injection of the dispersed phase was completed, the organic solvent was removed while maintaining the suspension temperature at 40°C for 3 hours. Subsequently, the temperature was cooled to 25°C, the microspheres were filtered, treated for removing residual polyvinyl alcohol by using tertiary distilled water, and lyophilized.

### Preparation Examples 5 to 32: Preparation of biodegradable polymer microsphere containing semaglutide or a pharmaceutically acceptable salt thereof

0.125 to 0.450 g of semaglutide as a drug (manufacturer: Chengdu Kaijie Biopharm Co., Ltd, China) and 0 to 1.2 g of a bioavailability enhancer (see Table 1) were used, and finally, the biodegradable polymer was weighed using at least one of R203H, RG203H, RG503H, RG653H, RG753H, PDL04A, and PDLG7504A in the range of 0.75 to 2.55 g, so that the batch size was 1.0 to 3.0 g (in Preparation Examples 1 to 32, Preparation Examples 16, 19, and 30 have a batch size of 3.0g, Preparation Examples 1, 13, 14, 15, 29, and 30 have a batch size of 2.0g, and the remainder have a batch size of 1.0g.). Dichloromethane was used as a solvent to prepare the dispersed phase, and Preparation Examples 4 to 29 and Preparation Examples 31 and 32 were homogeneously dissolved using at least one of glacial acetic acid and methanol as a co-solvent. The primary emulsion was prepared by dispersing the aqueous phase in the organic phase using a 40 µm porous emulsion membrane. In Preparation Examples 2 to 32, 2,000 ml of an aqueous solution containing 0.1% of polyvinyl alcohol (viscosity: 4.8~5.8 mPa.s) and 2.0% of Na₂HPO₄ (disodium phosphate) were used as the continuous phase, and in Preparation Example 1, 2,000 ml of an aqueous solution containing polyvinyl alcohol (viscosity: 4.8~ 5.8 mPa.s) and 1.0% of NaCl were used. The continuous phase was connected to an emulsification device equipped with a porous membrane with a diameter of 40 µm, and the prepared dispersed phase was injected into the porous membrane together with the continuous phase. An emulsion having dispersed biodegradable polymer microdroplets containing semaglutide was prepared, and the suspension was placed in a preparation container and stirred at a speed of 200 to 300 rpm.

The temperature of the preparation container was maintained at 25°C, and when the injection of the dispersed phase was completed, the organic solvent was removed while maintaining the suspension temperature at 40°C for 3 hours. Subsequently, the temperature was cooled to 25°C, the microspheres were filtered, treated for removing residual polyvinyl alcohol by using tertiary distilled water, and lyophilized.

Preparation Examples 5 and 10 did not include an initial-release inhibitor in the continuous phase.

**[Table 1]**

| Preparati on Example No. | Type of polymer | Used amount of polymer (g) | Used amount of semaglutid e (g) | Type /used amount of bioavailability enhancer (g) | Solvent | Co-solvent | Initial-release inhibitor and concentratio n |
|---|---|---|---|---|---|---|---|
| Preparati on Example 1 _{a) d)} | RG653H:R G753H=1:1 | 1.52 | 0.150 | Poloxamer F127 / 0.33 | DCM | - | 1% NaCl |
| Preparati on Example 2 _{a) d)} | PDL04A | 0.78 | 0.200 | Disodium phosphate / 0.02 | DCM | - | 2% Na₂HPO₄ |
| Preparati on Example 3 _{b) d)} | PDLG7504 A | 0.76 | 0.200 | Sodium Decanoate / 0.02 , Disodium phosphate / 0.02 | DCM | - | 2% Na₂HPO₄ |
| Preparati on Example 4 _{b) c)} | PDLG7504 A | 0.79 | 0.200 | Benzathine / 0.006 | DCM | DCM:acetic acid:methanol= 16:4:1 | 2% Na₂HPO₄ |
| Preparati on Example 5_{b) c)} | PDLG7504 A | 0.80 | 0.200 | N/A | DCM | DCM:acetic acid:methanol =16:4:1 | 2.5% NaCl |
| Preparati on Example 6 _{b) c)} | PDLG7504 A | 0.80 | 0.200 | N/A | DCM | DCM:acetic acid:methanol= 16:4:1 | 2% Na₂HPO₄ |
| Preparati on Example 7 _{b) c)} | PDLG7504 A | 0.79 | 0.200 | Benzathine / 0.012 | DCM | DCM:acetic acid:methanol =16:4:1 | 2% Na₂HPO₄ |
| Preparati on Example 8 _{b) c)} | PDLG7504 A | 0.78 | 0.200 | Benzathine / 0.023 | DCM | DCM:acetic acid:methanol= 16:4:1 | 2% Na₂HPO₄ |
| Preparati on Example 9 _{b) c)} | PDLG7504 A | 0.75 | 0.200 | Benzathine / *0.05* | DCM | DCM:acetic acid:methanol= 16:4:1 | 2% Na₂HPO₄ |
| Preparati on Example 10 _{b) c)} | RG653H:R G753H=1:1 | 0.80 | 0.200 | N/A | DCM | glacial acetic acid | 2.5% NaCl |
| Preparati on Example 11 _{b) c)} | RG503H:R G653H=1:1 | 0.86 | 0.125 | Benzathine / 0.012 | DCM | glacial acetic acid | 2% Na₂HPO₄ |
| Preparati on Example 12 _{b) c)} | RG653H:R G753H=1:1 | 0.84 | 0.150 | Benzathine / 0.012 | DCM | glacial acetic acid | 2% Na₂HPO₄ |
| Preparati on Example 13 _{b) c)} | RG653H:R G753H=1:1 | 1.63 | 0.350 | Benzathine / 0.024 | DCM | glacial acetic acid | 2% Na₂HPO₄ |
| Preparati on Example 14 _{b) c)} | RG653H:R G753H=1:1 | 1.58 | 0.400 | Benzathine / 0.024 | DCM | glacial acetic acid | 2% Na₂HPO₄ |
| Preparati on Example 15 _{b) c)} | RG653H:R G753H=1:1 | 1.60 | 0.400 | N/A | DCM | glacial acetic acid | 2% Na₂HPO₄ |
| Preparati on Example 16 _{b) c)} | R203H | 2.55 | 0.450 | N/A | DCM | glacial acetic acid | 2% Na₂HPO₄ |
| Preparati on Example 17 _{b) c)} | RG653H:R G203H=1:2 | 0.85 | 0.150 | N/A | DCM | glacial acetic acid | 2% Na₂HPO₄ |
| Preparati on Example 18_{b) c)} | RG503H | 0.84 | 0.150 | Benzathine / 0.012 | DCM | glacial acetic acid | 2% Na₂HPO₄ |
| Preparati on Example 19_{b) c)} | RG653H | 2.51 | 0.450 | Benzathine / 0.036 | DCM | glacial acetic acid | 2% Na₂HPO₄ |
| Preparati on Example 20 _{b) c)} | RG753H | 0.84 | 0.150 | Benzathine / 0.012 | DCM | glacial acetic acid | 2% Na₂HPO₄ |
| Preparati on Example 21 _{b) c)} | RG653H:R G203H=1:2 | 0.84 | 0.150 | Benzathine / 0.012 | DCM | glacial acetic acid | 2% Na₂HPO₄ |
| Preparati on Example 22 _{b) c)} | RG653H:R 203H=3:1 | 0.84 | 0.150 | Benzathine / 0.012 | DCM | glacial acetic acid | 2% Na₂HPO₄ |
| Preparati on Example 23 _{b) c)} | RG653H:R 203H=1:1 | 0.84 | 0.150 | Benzathine / 0.012 | DCM | glacial acetic acid | 2% Na₂HPO₄ |
| Preparati on Example 24 _{b) c)} | RG653H:R 203H=1:3 | 0.84 | 0.150 | Benzathine / 0.012 | DCM | glacial acetic acid | 2% Na₂HPO₄ |
| Preparati on Example 25 _{b) c)} | RG653H:R 203H=3:1 | 0.80 | 0.200 | N/A | DCM | glacial acetic acid | 2% Na₂HPO₄ |
| Preparati on Example 26 _{b) c)} | RG653H:R 203H=1:1 | 0.80 | 0.200 | N/A | DCM | glacial acetic acid | 2% Na₂HPO₄ |
| Preparati on Example 27 _{b) c)} | RG653H:R 203H=1:3 | 0.80 | 0.200 | N/A | DCM | glacial acetic acid | 2% Na₂HPO₄ |
| Preparati on Example 28 _{b) c)} | RG653H:R 203H=1:1 | 0.80 | 0.200 | N/A | DCM | glacial acetic acid | 2% Na₂HPO₄ |
| Preparati on Example 29 _{b) c)} | RG653H:R 203H=1:1 | 0.85 | 0.150 | N/A | DCM | glacial acetic acid | 2% Na₂HPO₄ |
| Preparati on Example 30 _{b) c)} | RG653H:R G753H=1:1 | 2.55 | 0.450 | N/A | DCM | - | 2% Na₂HPO₄ |
| Preparati on Example 31 _{b) c)} | RG653H:R G753H=1:1 | 1.69 | 0.300 | Benzathine / 0.006 | DCM | glacial acetic acid | 2% Na₂HPO₄ |
| Preparati on Example 32 _{b) c)} | RG653H:R G753H=1:1 | 1.68 | 0.300 | Benzathine / 0.024 | DCM | glacial acetic acid | 2% Na₂HPO₄ |
| Preparati on Example 52_{a) d)} | 653H:753H =1:1 | 0.4 | 0.150 | N/A | DCM | - | 2.5% NaCl |
| Preparati on Example 53 _{a) d)} | 653H:753H =1:1 | 0.4 | 0.150 | HPbCD / 0.15 | DCM | - | 2.5% NaCl |
| Preparati on Example 54_{a) d)} | 653H:753H =1:1 | 0.4 | 0.150 | Sucrose / 0.04 | DCM | - | 2.5% NaCl |
| Preparati on Example 55 _{b) c)} | 653H:753H =1:1 | 0.42 | 0.150 | Benzathine / 0.006 | DCM | glacial acetic acid | - |
| Preparati on Example 56 _{b) c)} | 653H:753H =1:1 | 0.42 | 0.150 | Benzathine / 0.006 and 2% Na₂HPO₄ | DCM | glacial acetic acid | 2% Na₂HPO₄ |
| Preparati on Example 57 _{b) c)} | 653H:753H =1:1 | 0.85 | 0.150 | 2% K₂HPO₄ | DCM | glacial acetic acid | 2% K₂HPO₄ |
| Preparati on Example 58 _{b) c)} | 653H:753H =1:1 | 0.43 | 0.150 | 2% NaH₂PO₄ | DCM | glacial acetic acid | 2% NaH₂PO₄ |
| Preparati on Example 59 _{b) c)} | 653H:753H =1:1 | 0.43 | 0.150 | 0.5% Na₂HPO₄ | DCM | glacial acetic acid | 0.5% Na₂HPO₄ |
| Preparati on Example 60 _{b) c)} | 653H:753H =1:1 | 0.43 | 0.150 | 1% Na₂HPO₄ | DCM | glacial acetic acid | 1.0% Na₂HPO₄ |
| Preparati on Example 61 _{b) c)} | 653H:753H =1:1 | 0.43 | 0.150 | 3% Na₂HPO₄ | DCM | glacial acetic acid | 3.0% Na₂HPO₄ |
| Preparati on Example 62 _{b) c)} | 653H:753H =1:1 | 0.43 | 0.150 | 4% Na₂HPO₄ | DCM | glacial acetic acid | 4.0% Na₂HPO₄ |
| Preparati on Example 63 _{b) c)} | 653H:753H =1:1 | 0.43 | 0.075 | 2% Na₂HPO₄ | DCM | glacial acetic acid | 2% Na₂HPO₄ |
| Preparati on Example 64 _{b) c)} | 653H:753H =1:1 | 0.85 | 0.15 | 2% Na₂HPO₄ | DCM | glacial acetic acid | 2% Na₂HPO₄ |
| Preparati on Example 65_{b) c)} | 653H:753H =1:1 | 0.85 | 0.15 | 2% Na₂HPO₄ | DCM | glacial acetic acid | 2% Na₂HPO₄ |
| Preparati on Example 66 _{b) c)} | 653H:753H =1:1 | 0.85 | 0.15 | - | DCM | glacial acetic acid | - |
| Preparati on Example 67 _{b) c)} | 653H:753H =1:1 | 46.1 | 8.25 | Benzathine / 0.66 and 2% NaH₂PO₄ | DCM | glacial acetic acid | 2% Na₂HPO₄ |
| Preparati on Example 68 _{b) c)} | 653H:753H =1:1 | 3.4 | 0.6 | 2% Na₂CO₃ | DCM | glacial acetic acid | 2% Na₂CO₃ |
| Preparati on Example 69 _{b) c)} | 653H:753H =1:1 | 0.85 | 0.15 | 2% NaHCO₃ | DCM | glacial acetic acid | 2% NaHCO₃ |
| Preparati on Example 70 _{b) c)} | 653H:753H =1:1 | 0.85 | 0.15 | 2% (NH₄)₂SO₄ | DCM | glacial acetic acid | 2% (NH₄)₂SO₄ |
| Preparati on Example 71 _{b) c)} | 653H:753H =1:1 | 0.85 | 0.15 | 2% (NH₄)₂HPO₄ | DCM | glacial acetic acid | 2% (NH₄)₂HPO₄ |

| | | | | | | | |
|---|---|---|---|---|---|---|---|
| a) Semaglutide sodium salt, b) Semaglutide free base, c) O/W method, d) W/O/W method | | | | | | | |

Preparation Examples 1 to 4 show the preparation of microsphere depending on different bioavailability enhancers by using poloxamer F127, disodium phosphate, sodium decanoate and disodium phosphate, and benzathine as bioavailability enhancers, respectively. Preparation Examples 4 to 9 show the preparation of microsphere depending on the content of benzathine, Preparation Examples 11 to 15 show the preparation of microsphere depending on the content of semaglutide, and Preparation Examples 16 to 29 show the preparation of microspheres depending on the polymer.

### Preparation Examples 33 to 37 (O/W method): Preparation of biodegradable polymer microsphere containing dexamethasone acetate or a pharmaceutically acceptable salt thereof

For the preparation of dexamethasone microsphere, the dispersed phase includes at least one of Purasorb PDLG 7502A (i.v 0.16-0.24 dl/g; manufacturer: Purac, Netherlands), RG 752H (i.v 0.14-0.22 dl/g; manufacturer: Evonik, Germany) and RG 753H (i.v 0.32-0.44 dl/g; manufacturer: Evonik, Germany), PDL04A, PDL02, PDLG 7504A (manufacturer: Corbion, Netherlands), which are biocompatible polymers, and 0.200 to 0.400 g of dexamethasone acetate (manufacturer: Pfizer, USA) was mixed with dichloromethane (manufacturer: J.T Baker, USA), dimethyl sulfoxide (manufacturer: J.T Baker, USA), and benzyl alcohol (manufacturer: Junsei, Japan) to prepare a microsphere. In Preparation Examples 33 to 37, 2,000 ml of an aqueous solution containing 0.5% polyvinyl alcohol (viscosity: 4.8 to 5.8 mPa.s) and 2.5% of NaCl were used as the continuous phase.

**[Table 2]**

| Preparation Example No. | Type of polymer | Used amount of polymer (g) | Used amount of Dexamethasone (g) | Solvent | Co-solvent |
|---|---|---|---|---|---|
| Preparation Example 33 | 7502A:PDL04A=1:4 | 0.80 | 0.200 | DCM | benzyl alcohol |
| Preparation Example 34 | 7502A:RG752H:RG75 3H =44:9:20 | 0.73 | 0.268 | DCM | benzyl alcohol |
| Preparation Example 35 | 7502A:7504A=1:1 | 0.60 | 0.400 | DCM | benzyl alcohol |
| Preparation Example 36 | PDL02 | 0.60 | 0.400 | DCM | benzyl alcohol |
| Preparation Example 37 | PDL04A | 0.60 | 0.400 | DCM | benzyl alcohol |

Preparation Examples 33 to 37 show the preparation of microspheres depending on the type of polymer and the used amount of dexamethasone acetate.

### Preparation Examples 38 to 51

The co-administered formulation of semaglutide microsphere and dexamethasone acetate microsphere are shown as Preparation Examples 38 to 51 (see Table 3).

**[Table 3]**

| | Semaglutide microsphere | Dexamethasone microsphere | API ratio of semaglutide: dexamethasone | Ratio of semaglutide microsphere: dexamethasone microsphere | Semaglutide content when used in combination |
|---|---|---|---|---|---|
| Preparation Example 38 | Preparation Example 12 | Preparation Example 35 | 200 : 1 | 533 : 1 | 12.9 |
| Preparation Example 39 | Preparation Example 13 | Preparation Example 35 | 200 : 1 | 457 : 1 | 16.4 |
| Preparation Example 40 | Preparation Example 14 | Preparation Example 35 | 200 : 1 | 400 : 1 | 17.1 |
| Preparation Example 41 | Preparation Example 15 | Preparation Example 35 | 200 : 1 | 400 : 1 | 13.0 |
| Preparation Example 42 | Preparation Example 28 | Preparation Example 36 | 200 : 1 | 400 : 1 | - |
| Preparation Example 43 | Preparation Example 29 | Preparation Example 36 | 200 : 1 | 533:1 | - |
| Preparation Example 44 | Preparation Example 16 | Preparation Example 37 | 22 : 1 | 59 : 1 | 10.4 |
| Preparation Example 45 | Preparation Example 21 | Preparation Example 33 | 22 : 1 | 30 : 1 | 12.7 |
| Preparation Example 46 | Preparation Example 30 | Preparation Example 34 | 67 : 1 | 40 : 1 | 13.1 |
| Preparation Example 47 | Preparation Example 31 | Preparation Example 34 | 67 : 1 | 40 : 1 | 13.0 |
| Preparation Example 48 | Preparation Example 32 | Preparation Example 34 | 67 : 1 | 40 : 1 | 14.0 |
| Preparation Example 49 | Preparation Example 15 | Preparation Example 34 | 67 : 1 | 30 : 1 | 15.7 |
| Preparation Example 50 | Preparation Example 22 : Preparation Example 24 = 1:1 | Preparation Example 36 | 200 : 1 | 533 : 1 | - |
| Preparation Example 51 | Preparation Example 25 : Preparation Example 27 = 1:1 | Preparation Example 36 | 200 : 1 | 400 : 1 | - |

### Experimental Example 1: Measurement of the encapsulated amount of semaglutide in the microsphere

In order to measure the amount of encapsulated semaglutide in the microsphere prepared in Preparation Examples 1 to 32 and the amount of encapsulated dexamethasone in the microsphere prepared in Preparation Examples 33 to 37, 10 mg of microspheres were completely dissolved in DMSO, and then diluted with a mobile phase. 20 µL of the diluted solution was injected into HPLC and measured at a detection wavelength of 214 nm. The column used in this experiment was ZORBAX 300SB-C18, 5µm, 4.6x150 mm, and the mobile phase was a mixture of 45% of 0.1% TFA acetonitrile and 55% of 0.1% TFA aqueous solution in isocratic mode. The measured amount of encapsulated drug is shown in Table 4 below.

**[Table 4]**

| Formulation | Target loading amount (% w/w) | Drug content (% w/w) |
|---|---|---|
| Preparation Example 1 | 7.5 | 6.67 |
| Preparation Example 2 | 20 | 14.50 |
| Preparation Example 3 | 20 | 17.90 |
| Preparation Example 4 | 20 | 18.20 |
| Preparation Example 5 | 20.0 | 18.0 |
| Preparation Example 6 | 20.0 | 20.1 |
| Preparation Example 7 | 20.0 | 17.1 |
| Preparation Example 8 | 20.0 | 17.7 |
| Preparation Example 9 | 20.0 | 18.0 |
| Preparation Example 10 | 20.0 | 19.7 |
| Preparation Example 11 | 12.5 | 11.6 |
| Preparation Example 12 | 15.0 | 12.9 |
| Preparation Example 13 | 17.5 | 16.5 |
| Preparation Example 14 | 20.0 | 17.1 |
| Preparation Example 15 | 20.0 | 16.0 |
| Preparation Example 16 | 15.0 | 10.5 |
| Preparation Example 17 | 15.0 | 13.1 |
| Preparation Example 18 | 15.0 | 13.1 |
| Preparation Example 19 | 15.0 | 13.6 |
| Preparation Example 20 | 15.0 | 13.1 |
| Preparation Example 21 | 15.0 | 13.1 |
| Preparation Example 30 | 15.0 | 13.3 |
| Preparation Example 31 | 15.0 | 13.3 |
| Preparation Example 32 | 15.0 | 14.2 |
| Preparation Example 33 | 20.0 | 22.1 |
| Preparation Example 34 | 26.8 | 25.2 |
| Preparation Example 35 | 40.0 | 37.1 |
| Preparation Example 36 | 40.0 | 43.9 |
| Preparation Example 37 | 40.0 | 40.1 |
| Preparation Example 52 | 7.5 | 6.89 |
| Preparation Example 53 | 7.5 | 7.53 |
| Preparation Example 54 | 7.5 | 7.01 |
| Preparation Example 55 | 15.0 | 14.6 |
| Preparation Example 56 | 15.0 | 13.4 |
| Preparation Example 57 | 15.0 | 12.4 |
| Preparation Example 58 | 15.0 | 12.9 |
| Preparation Example 59 | 15.0 | 12.0 |
| Preparation Example 60 | 15.0 | 12.1 |
| Preparation Example 61 | 15.0 | 12.3 |
| Preparation Example 62 | 15.0 | 11.3 |
| Preparation Example 63 | 15.0 | 11.7 |
| Preparation Example 64 | 15.0 | 12.6 |
| Preparation Example 65 | 15.0 | 12.8 |
| Preparation Example 66 | 15.0 | 13.8 |
| Preparation Example 67 | 15.0 | 13.5 |
| Preparation Example 68 | 15.0 | 12.0 |
| Preparation Example 69 | 15.0 | 11.8 |
| Preparation Example 70 | 15.0 | 13.6 |
| Preparation Example 71 | 15.0 | 12.5 |

### Experimental Example 2: In-vivo pharmacokinetic test using rats

### Experimental Example 2-1: Change in blood concentration of semaglutide depending on bioavailability enhancer and release controlling agent

In order to evaluate the drug release profile in the body of the semaglutide sustained-release microsphere according to the present disclosure, the blood concentration of semaglutide was measured after administration to rats.

The microspheres were measured so as to the amount of semaglutide be 1.2 mg (4.0 mg/kg) or 3.6 mg (12.0 mg/kg), dispersed in 0.5 mL suspension, and then injected into SD rats (Sprague-Dawley Rat, 300 g). 0.5 mL of blood was collected at pre-planned times, and the blood concentration of semaglutide was measured using HPLC. The measured AUC and Cₘₐₓ are shown in Table 5 below.

**[Table 5]**

| Category | Dose (mg/head) | Dose (mg/kg) | AUC total (ng*day/mL )/(mg/mL) | Cₘₐₓ (ng/mL) | Dose-normalized AUC total (ng*day/m L)/(mg/kg) | Dose-normalized Cₘₐₓ (ng/mL)/(m g/kg) | AUC (%)₀₋₂₄ₕᵣₛ |
|---|---|---|---|---|---|---|---|
| Preparation Example 01 | 3.6 | 12 | 14217 | 2426.7 | 1184.8 | 202.2 | 9.2 |
| Preparation Example 02 | 1.2 | 4 | 2564 | 405.3 | 641.0 | 101.3 | 8.3 |
| Preparation Example 03 | 3.6 | 12 | 11490.0 | 1139.7 | 957.5 | 95.0 | 6.6 |
| Preparation Example 04 | 3.6 | 12 | 21564 | 1940.0 | 1815 | 161.7 | 8.9 |

As can be seen in Table 5, the microsphere containing semaglutide prepared in Preparation Examples 1 to 4 of the present disclosure, shows a high AUC and less than 10 % of the cumulative AUC ratio of from administration time to 24 hours after adminstration, which can represent the initial releasing profile at the time of administration.

### Experimental Example 2-2. Improvement of semaglutide bioavailability by the content of benzathine

In order to evaluate the drug release profile in the body of the semaglutide sustained-release microsphere according to the present disclosure, the bioavailability (%) after administration to rats was measured. Specifically, the content of benzathine relative to the total weight of the batch was set at 0.6 (w/w)%, 1.2 (w/w)%, 2.3 (w/w)%, and 5 (w/w)%, respectively. For a semaglutide sustained-release microsphere to which benzathine was added, and a semaglutide sustained-release microsphere to which benzathin was not added, 1.2 mg (4.0 mg/kg) or 3.6 mg (12.0 mg/kg) of semaglutide contained in the sustained-release microsphere were dispersed in 0.5 mL suspension, and then injected into SD rats (Sprague-Dawley Rat, 300 g). After that, in order to compare the improvement in bioavailability in rats, the pharmacokinetic parameters were analyzed. As a result, it was confirmed that Preparation Examples 4, 7, 8, and 9 showed an increase in AUC of about twice or more as compared to Preparation Example 6. It was confirmed that overall, the AUC was similar, and the bioavailability was higher than 40% or more.

**[Table 6]**

| Category | Bioavailability enhancer | Used amount of bioavailability enhancer (g) | Weight ratio of bioavailability enhancer/ semaglutide (w/w(%)) | AUCnorm. (ng*day/ml)/(mg /kg) |
|---|---|---|---|---|
| Preparation Example 4 | Benzathine | 0.006 | 3 | 1815.0 |
| Preparation Example 6 | None | N/A | - | 959.4 |
| Preparation Example 7 | Benzathine | 0.012 | 6 | 1861.0 |
| Preparation Example 8 | Benzathine | 0.023 | 11.5 | 1721.0 |
| Preparation Example 9 | Benzathine | 0.050 | 25 | 2026.0 |

### Experimental Example 2-3. Improvement of AUC by semaglutide content

In order to evaluate the in vivo drug releasing profile of semaglutide sustained-release microsphere according to the present disclosure, the AUC was measured after administration to rats.

In the experiment, the semaglutide sustained-release microspheres containing 0.125, 0.150, 0.350, and 0.400 g of semaglutide, respectively, and 1.2 g of benzathine as a bioavailability enhancer, and semaglutide sustained-release microspheres containing 0.400 g of semaglutide and no bioavailability enhancer were injected into rats. For injection of the semaglutide microspheres, the sustained-release microspheres containing 3.6 mg (12.0 mg/kg) as semaglutide was dispersed in 0.5 mL suspension and then injected into SD rats (Sprague-Dawley Rat, 300 g).

**[Table 7]**

| Category | Bioavailabili ty enhancer | Used amount of semaglutide (g) | Content of semaglutide (w/w)% | AUCnorm. (ng*day/ml)/(mg/kg) | AUCnorm. 0-28d (ng*day/mL)/(mg/ kg) |
|---|---|---|---|---|---|
| | | | | | |
| Preparation Example 12 | benzathine | 0.150 | 15.0 | 1379.6 | 1016.0 |
| Preparation Example 14 | benzathine | 0.400 | 20.0 | 3572.8 | 3296.6 |

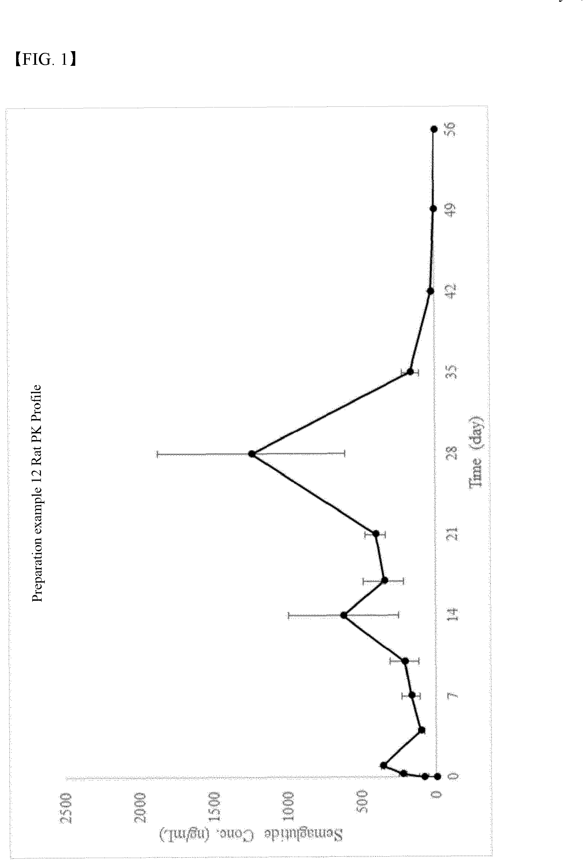

It was confirmed that AUC increased as the drug content increased. In particular, when the microspheres of Preparation Example 12 were administered to an experimental animal, the cumulative drug release rate within 28 days after administration was 62.28% of the total drug release amount according to the results of Fig. 1 showing the change in blood drug concentration and the pharmacokinetic parameters. These confirm that the ratio of the drug release amount within the drug administration period relative to the total drug release amount was 80% or less, showing a preferable drug release profile.

### Experimental Example 2-4. Initial release depending on whether initial-release inhibitor is used or not

Table 8 below shows the initial release depending on whether or not an initial-release inhibitor is included in the continuous phase.

**[Table 8]**

| | Initial-release inhibitor | Initial release |
|---|---|---|
| Preparation Example 10 | N/A | 9.1 |
| Preparation Example 15 | Disodium phosphate | 1.5 |
| Preparation Example 5 | N/A | 40.8 |
| Preparation Example 6 | Disodium phosphate | 1.9 |

As a result of the experiment, it was confirmed that the initial release of the drug in the microsphere containing a high content of semaglutide was significantly reduced becuase the initial-release inhibitor was contained in the continuous phase during the preparation of microspheres.

### Experimental Example 3-1. Comparison of microsphere properties by the

### addition of disodium phosphate and the use of similar salts

This experiment was carried out using the salts like disodium phosphate as the initial-release inhibitor to compare their characteristics during the preparation of microparticles.

A control formulation without addition of the initial release inhibitor in Table 1 (Preparation Example 55) and formulations prepared by adding Na₂HPO₄, K₂HPO₄, and NaH₂PO₄ as initial-release inhibitor were shown as Preparation Examples 56 to 58. Table 9 shows the main characteristics and initial release of Preparation Examples 55 to 58.

**[Table 9]**

| Preparation Example No. | Preparation Example 55 | Preparation Example 56 | Preparation Example 57 | Preparation Example 58 |
|---|---|---|---|---|
| Initial-release inhibitor | N/A | 2% Na₂HPO₄ | 2% K₂HPO₄ | 2% NaH₂PO₄ |
| Initial release (%) | 4.6 | 0.4 | 0.9 | 17.0 |
| pH | ≒ 3 | ≒ 7 | ≒ 7 | ≒ 5 |

Referring to Preparation Examples 55 to 58 of Tables 1 and 9, when Na₂HPO₄ or K₂HPO₄ was added as an initial-release inhibitor, the initial release decreased to less than 1% as compared to the control formulation. However, when NaH₂PO₄ was added to the continuous phase as an initial-release inhibitor, the initial release was 17%, which appeared to be very higher than that of the control formulation. These confirmed that even the salts similar to disodium phosphate did not show the effect of suppressing initial release. Preparation Example 55 had about pH 3, Preparation Examples 56 and 57 had about pH 7, Preparation Example 58 had about pH 5. Although it does not show linear data, the reason of high initial release in Preparation Examples 55 and 58 may be considered as a pH factor. When the cross-sectional image was observed with SEM, a large number of internal pores were distributed in the formulation with acidic pH, and conversely, the internal pores were reduced in the formuation with neutral or alkaline pH. The result was considered to explain the reason of suppressed initial release.

### Experimental Example 3-2. Comparison of microsphere characteristics by the type of initial-release inhibitor added

This experiment was carried out to compare the characteristics due to the type of an initial-release inhibitor when preparing the microsphere using Na₂CO₃, NaHCO₃, (NH₄)₂SO₄, and (NH₄)₂HPO₄ as an initial-release inhibitor.

A control formulation without addition of the initial-release inhibitor was shown in Table 1 (Preparation Example 66) and the formulations prepared by adding Na₂CO₃, NaHCO₃, (NH₄)₂SO₄, and (NH₄)₂HPO₄ as an initial-release inhibitor, respectively, were shown as Preparation Examples 68 to 71. Table 10 below, shows the main characteristics and the initial release of Preparation Examples 66 and 68 to 71.

**[Table 10]**

| Preparation Example No. | Preparation Example 66 | Preparation Example 68 | Preparation Example 69 | Preparation Example 70 | Preparation Example 71 |
|---|---|---|---|---|---|
| Initial-release inhibitor | N/A | 2(w/v)% Na₂CO₃ | 2(w/v)% NaHCO₃ | 2(w/v)% (NH₄)₂SO₄ | 2(w/v)% (NH₄)₂HPO₄ |
| Initial release (%) | 8.2 | 1.4 | 1.0 | 8.1 | 2.5 |
| pH | ≒ 3 | ≒ 12 | ≒ 8 | ≒ 5 | ≒ 7 |

Referring to Preparation Examples 66, 68 to 71 of Tables 1 and 10, the initial release appeared to be slightly higher in Preparation Examples 66 and 70. Preparation Examples 66 and 70 having high initial release showed acidic pH values of about 3 and about 5, respectively, and Preparation Examples 68, 69 and 71 with neutral and basic pH values exhibited less than 3% of an initial release. Based on these results, when the continuous phase exhibited an acidic pH by addition of an initial-release inhibitor to a continuous phase, the initial release of semaglutide appeared to be significantly high. However, when using an initial-release inhibitor that causes basic pH to appear at pH 7 or higher (neutral or basic), the initial release of semaglutide was clearly suppressed. Here, by considering the initial release of Preparation Examples 68, 69, and 71, the initial release was suppressed more when it was basic than when it was neutral, but the suppression of initial release did not increase linearly depending on the degree of basicity.

### Experimental Example 3-3. Comparison of microsphere characteristics by pH adjustment after addition of disodium phosphate

When the pH was intentionally adjusted after adding 2(w/v)% of disodium phosphate as an initial-release inhibitor, the test result of the change in initial release confirmed that the effect of suppressing the initial release on the microsphere was maintained even if the pH was changed after adding the initial-release inhibitor. Specifically, the control formulation (Preparation Example 66) without addition of the initial-release inhibitor in Table 1 and the formulation that 2 (w/v)% of disodium phosphate was added as an initial-release inhibitor but pH was not intentionally adjusted was shown as Preparation Example 63, the formulation in which the pH was adjusted to pH 2 (acidic) using HCl was shown as Preparation Example 64, and the formulation in which the pH was adjusted to pH 12 using NaOH was shown as Preparation Example 65.

Table 11 below shows the main characteristics and initial release of Preparation Examples 63 to 66.

**[Table 11]**

| Preparation Example No. | Preparation Example 63 | Preparation Example 64 | Preparation Example 65 | Preparation Example 66 |
|---|---|---|---|---|
| Initial-release inhibitor | 2(w/v)% Na₂HPO₄ | 2(w/v)% Na₂HPO₄ | 2(w/v)% Na₂HPO₄ | N/A |
| initial release (%) | 0.86 | 0.7 | 0.8 | 8.2 |
| pH | ≒ 8 | ≒ 2 | ≒ 12 | ≒ 3 |
| Remarks | | Adjusting pH after adding Na₂HPO₄ (using HCl) | Adjusting pH after adding Na₂HPO₄ (using NaOH) | |

As could be seen in Table 11, when the microspheres were prepared using a continuous phase containing 2 (w/v)% of Na₂HPO₄ as an initial-release inhibitor, the effect of suppressing the initial release of semaglutide was maintained, even if the pH of the continuous phase was intentionally adjusted to acidic at a later time.

### Experimental Example 4. Comparison of microsphere characteristics depending on the used amount of disodium phosphate

This experiment was carried out to compare the characteristics of microspheres depending on the amount of disodium phosphate added as an initial-release inhibitor.

A control formulation without addition of Na₂HPO₄ in Table 1 (Preparation Example 55) and formulations containing 0.5(w/v)%, 1(w/v)%, 2(w/v)%, 3(w/v)%, and 4(w/v)% of Na₂HPO₄ were shown as Preparation Examples 59, 60, 56, 61 and 62, respectively. Table 12 below shows the main characteristics and initial release of Preparation Examples 55, 56 and 59 to 62.

**[Table 12]**

| Preparation Example No. | Preparation Example 55 | Preparation Example 59 | Preparation Example 60 | Preparation Example 56 | Preparation Example 61 | Preparation Example 62 |
|---|---|---|---|---|---|---|
| initial-release inhibitor | N/A | 0.5(w/v)% Na₂HPO₄ | 1(w/v)% Na₂HPO₄ | 2(w/v)% Na₂HPO₄ | 3 (w/v)% Na₂HPO₄ | 4(w/v)% Na₂HPO₄ |
| initial release (%) | 4.6 | 0.64 | 0.56 | 0.4 | 0.77 | 0.86 |

Referring to Preparation Examples 55, 56, and 59 to 62 in Tables 1 and 12, the control formulation had 4.6 % of the initial release, whereas the initial release of the formulation containing 0.5 (w/v)% of Na₂HPO₄ decreased to 0.64%. When the Na₂HPO₄ concentration was 1 (w/v)% or 2(w/v)%, the initial release rates were confirmed to be lowered to 0.56% and 0.4%, respectively. However, when 3 (w/v)% or 4 (w/v)% of Na₂HPO₄ was added, the initial release tended to increase to 0.77% and 0.86%, respectively, as compared to when 2% of Na₂HPO₄ was added. When Na₂HPO₄ was used at 4 (w/v)% or more, the polymer agglomeration phenomenon occured largely in the preparation of the microsphere. It was suggested that the tendency for initial release to increase as the Na₂HPO₄ content increased by starting from 2 (w/v)% was due to the polymer agglomeration phenomenon described above.

### Experimental Example 5. Method for measuring residual amount of sodium and phosphorus

In order to measure the content of sodium and phosphorus in the microsphere used in Examples and Comparative Examples, 6 mL of nitric acid aqueous solution containing 300mg of the microsphere mixed with ultrapure water at a ratio of 1:1 was mixed with 3 mL of hydrogen peroxide, and then heated at 100°C or more. Acid was added until the gas generated during the dissolution process changed from yellow to white. The specimen obtained thereby was weighed, dissolved in ultrapure water, and then injected into an inductively coupled plasma emission spectrometer (ICP-OES) (Thermo Scientific Co., iCAP 6300 Duo, UK), and measured at a detection wavelength of 598.5 nm. The results are shown in the following Experimental Examples 5-1 and 5-2.

### Experimental Example 5-1. Comparison of the residual amount of sodium and phosphorus in microspheres depending on the amount of disodium phosphate added

The residual amounts of sodium and phosphorus in the microsphere according to Preparation Examples 59 to 61 and 63 was measured and shown in Table 13 below.

**[Table 13]**

| Preparation Example No. | Preparation Example 59 | Preparation Example 60 | Preparation Example 63 | Preparation Example 61 |
|---|---|---|---|---|
| C.P Excipients | 0.5(w/v)% Na₂HPO₄ | 1(w/v)% Na₂HPO₄ | 2(w/v)% Na₂HPO₄ | 3(w/v)% Na₂HPO₄ |
| Residual sodium (mg/kg) | 21 | 67 | 103 | 166 |
| Residual phosphorus (mg/kg) | 6 | 14 | 32 | 55 |

Referring to Table 13, it was confirmed that as the added amount of Na₂HPO₄ increased, the residual amounts of sodium and phosphorus increased significantly. The residual content of the initial-release inhibitor in the microspheres was 10 ppm to 200 ppm based on sodium, and 5 ppm to 100 ppm based on phosphorus.

### Experimental Example 5-2. Comparison of the residual amount of sodium and phosphorus in microspheres depending on changes in biodegradable polymer and addition of benzathine

The residual amounts of Na and P in Preparation Examples 16, 63, and 67 were measured and shown in Table 14.

**[Table 14]**

| Preparation Example No. | Preparation Example 16 | Preparation Example 67 | Preparation Example 63 |
|---|---|---|---|
| C.P Excipients | 2(w/v)% Na₂HPO₄ | 2(w/v)% Na₂HPO₄ | 2(w/v)% Na₂HPO₄ |
| Additive | N/A | 1.2% benzathine | N/A |
| Polymer | 203H | 653H:753H | 653H:753H |
| Residual sodium (mg/kg) | 397 | 154 | 103 |
| Residual phosphorus (mg/kg) | 80 | 16 | 32 |

Referring to Table 14, it was confirmed that depending on the preparation conditions of the microspheres containing semaglutide, and the initial-release inhibitor contained in the continuous phase, the contents of residual sodium and residual phosphorus were different, and the residual content of the initial-release inhibitor in the microspheres was 50 ppm to 500 ppm based on sodium, and 10 ppm to 100 ppm based on phosphorus.

### Experimental Example 6. Comparison of SEM results of microspheres depending on whether or not disodium phosphate is used in the continuous phase

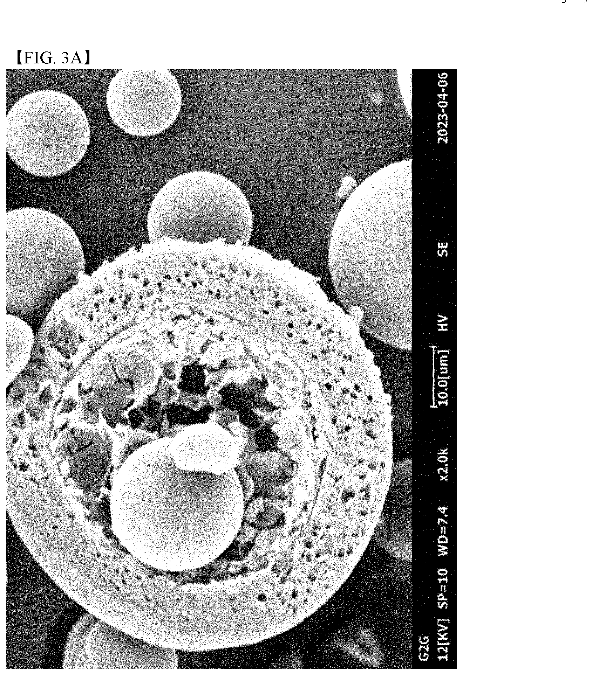

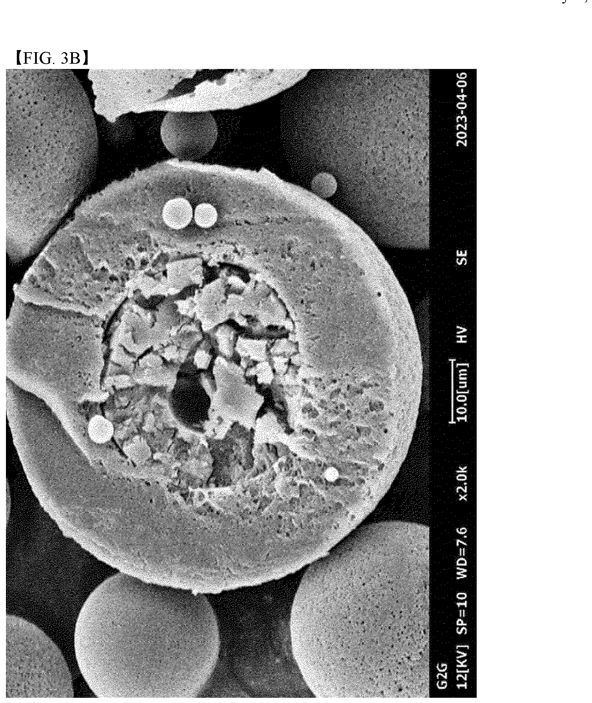

FIG. 3A shows the microspheres prepared using only 0.1 (w/v)% of PVA in the continuous phase, and FIG. 3B shows the microspheres prepared using 0.1 (w/v)% of PVA and 2 (w/v)% of Na₂HPO₄ in the continuous phase.

Referring to FIGs. 3A and 3B, it was confirmed that the microparticle prepared using only 0.1% of PVA in the continuous phase had many pores present in the cross section of the microsphere. It was confirmed that the microspheres prepared using 0.1 (w/v)% of PVA and 2 (w/v)% of Na₂HPO₄ in the continuous phase, most pores disappeared or that even if pores existed, the number and size of pores decreased significantly. This may be suggested to exhibit the effect of reducing the channels being capable of affecting the initial release of the drug.

## Claims

1. A pharmaceutical composition for preventing or treating diabetes, type 2 diabetes, preservation of beta-cell function, obesity, non-alcoholic steatohepatitis, or neurodegenerative disease, comprising a sustained-release microsphere consisting of semaglutide or a pharmaceutically acceptable salt thereof, a bioavailability enhancer and a biodegradable polymer,
wherein the semaglutide or a pharmaceutically acceptable salt thereof is contained in an amount of 8 wt% or more as semaglutide based on the total weight of the microsphere, and the bioavailability enhancer is contained in an amount of 2.5 wt% to 250 wt% based on the weight of semaglutide.

2. A pharmaceutical composition for preventing or treating diabetes, type 2 diabetes, preservation of beta-cell function, obesity, non-alcoholic steatohepatitis, or neurodegenerative disease, comprising a sustained-release microsphere consisting of semaglutide or a pharmaceutically acceptable salt thereof, an initial-release inhibitor and a biodegradable polymer,
wherein the semaglutide or a pharmaceutically acceptable salt thereof is contained in an amount of 8 wt% or more as semaglutide based on the total weight of the microspheres, and the initial-release inhibitor is contained at 5 ppm to 2,000 ppm.

3. The pharmaceutical composition according to claim 1 or 2, wherein the sustained-release microsphere releases less than 20% of semaglutide or a pharmaceutically acceptable salt thereof within 24 hours when administered in vivo.

4. The pharmaceutical composition according to claim 1 or 2, wherein the sustained-release microsphere releases less than 15% of semaglutide or a pharmaceutically acceptable salt thereof within 24 hours when administered in vivo.

5. The pharmaceutical composition according to claim 1 or 2, wherein the sustained-release microsphere releases less than 10% of semaglutide or a pharmaceutically acceptable salt thereof within 24 hours when administered in vivo.

6. The pharmaceutical composition according to claim 1 or 2, wherein the biodegradable polymer is at least one selected from the group consisting of: a polymer selected from the group consisting of polylactide (PLA), polyglycolide (PGA), polylactide-co-glycolide (PLGA), which is a copolymer of lactide and glycolide, polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); copolymers or simple mixtures of two or more thereof, a copolymer of the above polymer with polyethylene glycol (PEG); and a polymer-sugar conjugate in which the polymer or the copolymer is conjugated to sugar.

7. The pharmaceutical composition according to claim 1 or 2, wherein the pharmaceutically acceptable salt of the semaglutide is sodium salt, acetate, benzoate, hydroxynaphthoate, napadisilate, or pamoate of semaglutide.

8. The pharmaceutical composition according to claim 1, wherein the bioavailability enhancer is at least one selected from the group consisting of sodium decanoate, disodium phosphate, choline, meglumine, basic aluminum carbonate, dihydroxyaluminum sodium carbonate, ammonium phosphate, histidine, HEPES, HEPPS, spermine, spermidine, putrescine, methylene blue, proline, sugar, glycerol, surfactant, arginine, glycine, guanidine hydrochloride, urea, sodium chloride, potassium chloride, triethylamine, ethanolamine, triethanolamine, ethylenediamine, poloxamer, benzathine, procaine, lidocaine, bupivacaine, ropivacaine, oxytetracycline, sunitinib, rhizolutin, benzofuran, magnesium carbonate, magnesium hydroxide, magnesium oxide, magnesium trisilicate, zinc carbonate, zinc hydroxide, zinc phosphate, aluminum hydroxide, aluminum phosphate, dihydroxyaluminum aminoacetate, calcium phosphate, calcium hydroxide, magaldrate, and benzofuran derivatives.

9. The pharmaceutical composition according to claim 1, further comprising 5ppm to 2,000ppm of an initial-release inhibitor.

10. The pharmaceutical composition according to claim 2 or 9, wherein the initial-release inhibitor is at least one substance selected from alkali metal, alkaline earth metal or ammonium phosphate salt, phosphide salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt and nitride salt.

11. The pharmaceutical composition according to claim 1 or 2, wherein the sustained-release microsphere has an average particle size of 5 µm to 100 µm.

12. The pharmaceutical composition according to claim 1 or 2, wherein the weight of the sustained-release microspheres is 20 to 1,000 mg.

13. The pharmaceutical composition according to claim 1 or 2, wherein the biodegradable polymer has an intrinsic viscosity of 0.16 dL/g to 1.7 dL/g.

14. The pharmaceutical composition according to claim 1 or 2, further comprising one or more release controlling agents selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, naphadicylic acid, naphthalene sulfonic acid and pamoic acid.

15. A method for preparing a microsphere consisting of semaglutide or a pharmaceutically acceptable salt thereof, a bioavailability enhancer and a biodegradable polymer, the method comprising the steps of:
(a) dissolving semaglutide or a pharmaceutically acceptable salt thereof, a bioavailability enhancer, and one or more biodegradable polymers in an organic solvent to prepare a solution (dispersed phase) containing semaglutide or a pharmaceutically acceptable salt thereof, a bioavailability enhancer, and a polymer;
(b) adding the solution containing semaglutide or a pharmaceutically acceptable salt thereof, a bioavailability enhancer, and a polymer prepared in step (a) to an aqueous solution phase (continuous phase) containing a surfactant to prepare an emulsion;
(c) extracting the organic solvent from the dispersed phase in the emulsion state prepared in step (b) into a continuous phase and evaporating the organic solvent to form a microsphere; and
(d) recovering the microsphere from the continuous phase of step (c).

16. The method according to claim 15, wherein the bioavailability enhancer is at least one selected from the group consisting of: poloxamer, benzathine, procaine, lidocaine, bupivacaine, ropivacaine, oxytetracycline, sunitinib, rhizolutin and benzofuran.

17. A method for preparing a microsphere consisting of semaglutide or a pharmaceutically acceptable salt thereof, a bioavailability enhancer and a biodegradable polymer, the method comprising the steps of:
(a') dissolving semaglutide or a pharmaceutically acceptable salt thereof and a bioavailability enhancer in an aqueous solution to prepare a primary aqueous solution phase, dissolving one or more biodegradable polymers in an organic solvent to prepare an oil phase, and then mixing the primary aqueous solution and the oil phase to prepare a W/O emulsion (primary emulsion) containing semaglutide or a pharmaceutically acceptable salt thereof, a bioavailability enhancer, and a polymer;
(b') adding the primary emulsion containing semaglutide or a pharmaceutically acceptable salt thereof, a bioavailability enhancer, and a polymer prepared in step (a') to an aqueous solution phase (continuous phase) containing a surfactant to prepare a W/O/W emulsion (secondary emulsion);
(c') extracting the organic solvent from the oil phase in the secondary emulsion state prepared in step (b') into a continuous phase and evaporating the organic solvent to form a microsphere; and
(d') recovering the microsphere from the continuous phase of step (c').

18. The method according to claim 17, wherein the bioavailability enhancer is at least one selected from the group consisting of: sodium decanoate, disodium phosphate, choline, meglumine, basic aluminum carbonate, dihydroxyaluminum sodium carbonate, ammonium phosphate, histidine, HEPES, HEPPS, spermine, spermidine, putrescine, methylene blue, proline, sugar, glycerol, surfactant, arginine, glycine, guanidine hydrochloride, urea, sodium chloride, potassium chloride, triethyleneamine, ethanolamine, triethanolamine, and ethylenediamine.

19. A method for preparing a microsphere consisting of semaglutide or a pharmaceutically acceptable salt thereof, a bioavailability enhancer and a biodegradable polymer, the method comprising the steps of:
(a") dissolving semaglutide or a pharmaceutically acceptable salt thereof and one or more biodegradable polymers in an organic solvent, and suspending a bioavailability enhancer in this solution to prepare a suspension (dispersed phase) containing semaglutide or a pharmaceutically acceptable salt thereof, a bioavailability enhancer, and a polymer;
(b") adding the suspension containing semaglutide or a pharmaceutically acceptable salt thereof, a bioavailability enhancer, and a polymer prepared in step (a") to an aqueous solution phase (continuous phase) containing a surfactant to prepare an emulsion;
(c") extracting the organic solvent from the dispersed phase in the emulsion state prepared in step (b") into a continuous phase and evaporating the organic solvent to form a microsphere; and
(d") recovering the microsphere from the continuous phase of step (c").

20. The method according to claim 19, wherein the bioavailability enhancer is at least one selected from the group consisting of: magnesium carbonate, magnesium hydroxide, magnesium oxide, magnesium trisilicate, zinc carbonate, zinc hydroxide, zinc phosphate, aluminum hydroxide, aluminum phosphate, dihydroxyaluminum aminoacetate, calcium phosphate, calcium hydroxide, magaldrate, and benzofuran derivatives.

21. The method according to any one of claims 15 to 20, wherein the biodegradable polymer is at least one selected from the group consisting of: a polymer selected from the group consisting of polylactide (PLA), polyglycolide (PGA), polylactide-co-glycolide (PLGA), which is a copolymer of lactide and glycolide, polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly (butylene succinate lactide) (PBSLA); copolymers or simple mixtures of two or more thereof; a copolymer of the above polymer with polyethylene glycol (PEG); and a polymer-sugar conjugate in which the polymer or the copolymer is conjugated to sugar.

22. The method according to any one of claims 15 to 20, further comprising one or more release controlling agents selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, arachidic acid, isocrotonic acid, oleic acid, elaidic acid, sorbic acid, linoleic acid, arachidonic acid, hydroxynaphthoic acid, naphadicylic acid, and pamoic acid.

23. The method according to any one of claims 15 to 20, wherein the organic solvent in step (a) or (a') or (a") is at least one selected from the group consisting of dichloromethane, chloroform, ethyl acetate, methyl ethyl ketone, acetone, acetonitrile, dimethyl sulfoxide, dimethylformamide, N-methylpyrrolidone, acetic acid, methyl alcohol, ethyl alcohol, propyl alcohol and benzyl alcohol.

24. The method according to any one of claims 15 to 20, wherein the surfactant in step (b) or (b') or (b") is polyvinyl alcohol.

25. The method according to any one of claims 15 to 20, wherein the continuous phase in step (b) or (b') or (b") is water, or a mixed solvent of water and one or more selected from the group consisting of methyl alcohol, ethyl alcohol, propyl alcohol and ethyl acetate.

26. The method according to any one of claims 15 to 20, wherein the continuous phase in step (b) or (b') or (b") comprises an initial-release inhibitor.

27. The method according to claim 26, wherein the initial release inhibitor is contained in an amount of 0.05 w/v% to 20 w/v% based on the total volume of the continuous phase.

28. The method according to claim 26, wherein the initial release inhibitor is at least one selected from the group consisting of disodium phosphate, dipotassium phosphate, and diammonium phosphate.

29. The method according to any one of claims 15 to 20, wherein the continuous phase in step (b) or (b') or (b") has a pH of 7 or more.

30. The method according to any one of claims 15 to 20, wherein the release rate of semaglutide or a pharmaceutically acceptable salt thereof within 24 hours is less than 15%, when the prepared sustained-release microsphere containing semaglutide or a pharmaceutically acceptable salt thereof and a bioavailability enhancer is administered in vivo.

31. The method according to any one of claims 15 to 20, wherein the release rate of semaglutide or a pharmaceutically acceptable salt thereof within 24 hours is less than 10%, when the prepared sustained-release microsphere containing semaglutide or a pharmaceutically acceptable salt thereof and a bioavailability enhancer is administered in vivo.
